# EUROPEAN PATENT APPLICATION

(11) **EP 1 219 634 A1**
(43) Date of publication of application: **03.07.2002**
(21) Application number: 00128402.5
(22) Date of filing: 27.12.2000
(51) Int. Cl.: C07K 9/00, C07H 15/203, C07D 491/22, A61K 31/70, A61K 38/14

(54) **Cytostatic-glycoconjugates having specifically cleavable peptidic linking units**

(71) Applicant: Bayer Aktiengesellschaft, 51368 Leverkusen (DE)
(72) Inventor: Lerchen, Hans-Georg, Dr., 51375 Leverkusen (DE); Baumgarten, Jörg, Dr., 42115 Wuppertal (DE); Lockhoff, Oswald, Dr., 51373 Leverkusen (DE)

(57) **Abstract**

The present invention relates to cytostatics which have a tumor-specific action as a result of linkage to specific carbohydrate moeities via preferred linking units which can be selevtively cleaved by enzymes such as metallo matrixproteases (MMPs), elastase or cathepsines, i.e. by enzymes which can especially be found in tumor tissue. The preferred linking units guarantee sufficient serum stability of the conjugate of cytostatic and carbohydrate moeity and, at the same time, the desired intracellular action within tumour cells as a result of its specific enzymatic or hydrolytic cleavability with release of the cytostatic.

## Description

The present invention relates to cytostatics which have a tumor-specific action as a result of linkage to specific carbohydrate moeities via preferred linking units which can be selevtively cleaved by enzymes such as metallo matrixproteases (MMPs), i.e. by enzymes which can especially be found in tumor tissue. The preferred linking units guarantee sufficient serum stability of the conjugate of cytostatic and carbohydrate moeity and, at the same time, the desired intracellular action within tumour cells as a result of its specific enzymatic or hydrolytic cleavability with release of the cytostatic.

Chemotherapy in cancer is accompanied by usually serious side effects which are to be attributed to the toxic action of chemotherapeutics on proliferating cells of other tissue types than tumour tissue. For many years, scientists have occupied themselves with the problem of improving the selectivity of active compounds employed. A frequently followed approach is the synthesis of prodrugs which are released more or less selectively in the target tissue, for example, by change of the pH (Tietze et al., e.g. DE-A-4 229 903), by enzymes (e.g. glucuronidases; Jacquesy et al., EP-A-0 511 917; Bosslet et al., EP-A-0 595 133) or by antibody-enzyme conjugates (Bagshawe et al., WO 88/07378; Senter et al., US-4 975 278; Bosslet et al., EP-A-0 595 133). A problem in these approaches is, inter alia, the lack of stability of the conjugates in other tissues and organs, and in particular the ubiquitous active compound distribution which follows the extracellular release of active compound in the tumour tissue.

The marked lectin pattern on tumour cell surfaces (Gabius; Onkologie 12, (1989), 175) opens up the fundamental possibility of addressing these specifically on tumour cells by linkage of appropriate carbohydrate units to cytostatics. This prospect is restricted by the fact that, even in other tissues, in particular in the liver, lectins having similar carbohydrate specificities (galactose, lactose, mannose, N-acetylglucosamine, fucose etc.) occur (Ashwell et al., Annu. Rev. Biochem. 46 (1982), 531; Stahl et al. Proc. Natl. Acad. Sci. USA 74 (1977), 1521; Hill et al., J. Biol. Chem. 262 (1986), 7433; Jansen et al., J. Biol. Chem. 266 (1991), 3343). Accordingly, a marked concentration of active compound-containing glycoconjugates in the liver and other lectin-rich organs must be expected if, in this approach, carbohydrates are used without particular modification establishing a selectivity to tumour tissue.

The heterocyclic amine batracylin (1) shows a good antitumour action in various stomach cancer models (US-4 757 072).

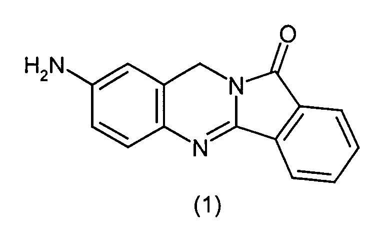

Peptide conjugates of (1) having good in-vitro action and more favourable solubility properties (US-4 180 343) are more poorly tolerable in animal experiments than free batracylin. The fucose conjugates of batracylin (1) described in EP-A-0 501 250 disadvantageously concentrate very strongly in the liver.

Quinolone-a (2), 7-[(3a-R,S, 4-R,S, 7a,S,R)-4-amino-1,3,3a,4,7,7a-hexahydro-iso-indol-2-yl]-8-chloro-1-cyclopropyl-6-fluoro-1,4-dihydro-4-oxo-3-quinolinecarboxylic acid, also shows, in addition to its outstanding antibacterial activity, a very good activity against various tumour cell lines (EP-A-0 520 240, JP-4 253 973). However, considerable toxicological problems face it (e.g. genotoxicity, bone marrow toxicity, high acute toxicity in vivo etc.).

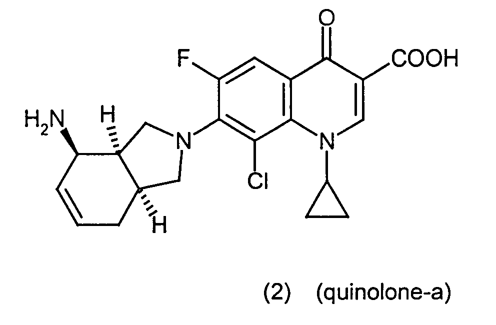

20(S)-Camptothecin is a pentacyclic alkaloid which was isolated in 1966 by Wall et al. (J. Am. Chem. Soc. 88, 3888 (1966)). It has a high active antitumour potential in numerous in-vitro and in-vivo tests. Unfortunately, however, the realization of the promising potential in the clinical investigation phase failed because of toxicity and solubility problems.

By opening of the E ring lactone and formation of the sodium salt, a water-soluble compound was obtained which is in a pH-dependent equilibrium with the ring-closed form. Here too, clinical studies have not led to success as yet.

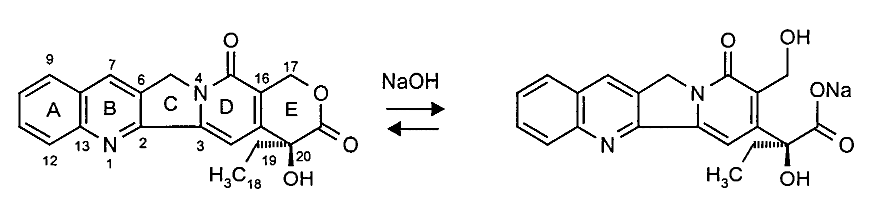

About 20 years later, it was found that the biological activity is to be attributed to enzyme inhibition of topoisomerase I. Since then, the research activities have again been increased in order to find a camptothecin derivative which is more tolerable and which is active in vivo.

For improvement of the water solubility, salts of A ring- and B ring-modified camptothecin derivatives and of 20-O-acyl derivatives with ionizable groups have been described (Vishnuvajjala et al. US 4 943 579). The latter prodrug concept was later also transferred to modified camptothecin derivatives (Wani et al. WO 9602546). The described 20-O-acyl prodrugs, however, have a very short half-life in vivo and are very rapidly cleaved to give the parent structure.

WO 96/31532 describes carbohydrate-modified cytostatics in which both serum stability and release of the cytostatic within the tumour cells and a specific concentration of the cytostatic in tumour tissue is achieved by a novel linkage of selectively modified carbohydrates to cytostatics (for example batracylin, quinolone-a, camptothecin) via preferred spacer and linker groups.

Further carbohydrate-modified cytostatics are described in WO 98/14459, WO 98/14468, WO 98/15571, WO 98/15573 and WO 98/51703.

Tumor cells have several strategies of surviving a treatment with cytotoxic drugs, which is the reason for the still existing need for new drugs for the treatment of cancer.

Fundamentally, the actvity of the above-mentioned glycoconjugates from the prior art should be improvable by designing the linker moeity combining the cytostatic radical with the carbohydrate radical such that it is cleavable by enzymes such as metallo matrixproteases (MMPs), i.e. by enzymes which can especially be found in tumor tissue. Such compounds should be capable of releasing the cyctostatic moeity even more selectively into or at the vicinity of tumor cells.

It was therefore the object of the present invention to develop conjugates which comprise a carbohydrate moiety and a cytostatic which can be released from the conjugate preferably at least in the vicinity of tumor tissue.

The above object is achieved by conjugates which comprise a carbohydrate moiety, a cytostatic and a linking unit which is selectively enzymatically cleavable with release of the cytostatic by enzymes such as metallo matrixproteases (MMPs), elastase or cathepsines, i.e. by enzymes which can especially be found in tumor tissue.

According to the present invention, the linking unit can be cleaved by tumour-associated enzymes. This leads to a further increase in the tissue specificity of the conjugates according to the invention and thus to an additional decrease of the conjugates according to the invention in other tissue types.

According to a further preferred embodiment of the invention, the linking unit can be cleaved by enzymes which are coupled to antibodies with selectivity for tumour tissue and are thus addressed to tumour tissue. This is also called the ADEPT approach. This likewise leads to a further increase in the tissue specificity of the conjugates according to the invention and thus to an additional decrease of the conjugates according to the invention in other tissue types.

Particularly preferred conjugates according to the present invention are those of the general formula (I)

CT - LI - Sp1 - Sp2 - K (I)

in which
- CT: denotes a cytotoxic radical or a radical of a cytostatic or of a cytostatic derivative, which can additionally carry a hydroxyl, carboxyl or amino group,
- LI: is a linker group comprising 5 to 8 amino acid residues in the D or L configuration, which can each optionally carry protective groups,
- Sp1: is absent or a carbonyl or a thiocarbonyl radical,
- Sp2: is an optionally substituted arylen or alkylen radical,
- K: is an unsubstituted or regioselectively modified carbohydrate radical;
and their physiologically acceptable salts, hydrates and stereoisomers.

Of the conjugates of the formula (I), according to a preferred embodiment those conjugates are preferred in which
- LI: is a linker group having the formula
-AA1-AA2-AA3-AA4-AA5-AA6-AA7-AA8-
wherein at least 5 of the radicals AA1 to AA8 are present, AA1 is bonded to the radical CT and
AA1 is a naturally occurring amino acid in the D or L configuration, which is selected from the group consisting of glycine, alanine, valine, leucine, isoleucine, histidine, glutamate, aspartate, serine, lysine, ornithine and phenylalanine;
AA2 is absent or is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine, and can optionally carry protective groups,
AA3 is absent or is a naturally occurring amino acid in the D or L configuration, which is selected from the group consisting of alanine, valine, phenylalanine, tyrosine, serine, isoleucine, lysine, glutamate, histidine, glycine, arginine, aspartate, tryptophane, proline, ornithine, methionine, S-methyl-cysteine, norvaline and leucine, and can optionally carry protective groups,
AA4 is absent or is a naturally occurring amino acid in the D or L configuration, which is selected from the group consisting of glycine, alanine, valine, leucine, isoleucine, cysteine and norvaline;
AA5 is absent or is a naturally occurring amino acid in the D or L configuration, which is selected from the group consisting of glycine, alanine, valine, leucine, isoleucine, histidine, tyrosine, glutamine, asparagine, proline, methionine, phenylalanine and cysteine;
AA6 is absent or is a naturally occurring amino acid in the D or L configuration, which is selected from the group consisting of glycine, alanine, valine, leucine, isoleucine, histidine, glutamine, asparagine, aspartate and proline;
AA7 is absent or is a naturally occurring amino acid in the D or L configuration, which is selected from the group consisting of glycine, alanine, valine, leucine, isoleucine, histidine, γ-aminobutyric acid, aspartate, glutamate, lysine and proline;
AA8 is absent or is a naturally occurring amino acid in the D or L configuration, which is selected from the group consisting of glycine, alanine, valine, leucine, isoleucine, histidine, lysine, proline and γ-aminobutyric acid;
and the other radicals CT, Sp1, Sp2 and K are as defined above.

### Particularly preferred are conjugates according to formula (I), in which

- LI: is a linker group having the formula
-AA1-AA2-AA3-AA4-AA5-AA6-AA7-AA8-
wherein 5 to 7 of the radicals AA1 to AA8 are present, AA1 is bonded to the radical CT and
AA1 is valine, glycine, leucine, histidine;
AA2 is absent or is a naturally occurring amino acid in the D or L configuration, which is selected from the group consisting of alanine, phenylalanine, serine, isoleucine, glutamate, asparagine, glutamine, histidine, glycine, aspartate, tryptophane, proline, and leucine, and can optionally carry protective groups,
AA3 is absent or is a naturally occurring amino acid in the D or L configuration, which is selected from the group consisting of alanine, phenylalanine, serine, isoleucine, norvaline, S-methyl-cysteine, methionine, glutamate, histidine, glycine, aspartate, tryptophane, and leucine, and can optinally carry protective groups,
AA4 is absent or is a naturally occurring amino acid in the D or L configuration, which is selected from the group consisting of glycine, leucine, cysteine and norvaline, and can optionally carry protective groups,
AA5 is absent or is a naturally occurring amino acid in the D or L configuration, which is selected from the group consisting of glycine, alanine, valine, leucine, histidine, glutamine, phenylalanine, isoleucine, and methionine,
AA6 is absent or is a naturally occurring amino acid in the D or L configuration, which is selected from the group consisting of glycine, proline, glutamine, methionine, and leucine;
AA7 is absent or is a naturally occurring amino acid in the D or L configuration, which is selected from the group consisting of glycine, leucine, aspartate, histidine, γ-aminobutyric acid and proline;
AA8 is absent or is a naturally occurring amino acid in the D or L configuration, which is selected from the group consisting of glycine, proline and γ-aminobutyric acid ;
and the other radicals CT, Sp1, Sp2 and K are as defined above.

Of the conjugates of the formula (I), according to a preferred embodiment those conjugates are preferred in which
- CT: is camptothecin or a camptothecin derivative, which can be bonded to the rest of the conjugate via the C20-OH group, or doxorubicine, or quinolone a;
- LI: is as defined above;
- Sp1: is absent, or is a carbonyl or a thiocarbonyl radical,
- Sp2: is an optionally substituted arylene or alkylene radical,
- K: is a carbohydrate moiety of the formula (II) 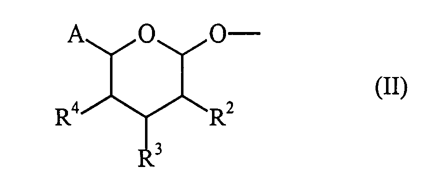 wherein
A is methyl, hydroxymethyl, carboxy and esters and amides derived therefrom, alkoxymethyl, acyloxymethyl oder carboxyalkyloxymethyl and esters and amides derived therefrom, or CH₂-B,
wherein
B is also a carbohydrate radical of the formula (II) which is bonded via its anomeric centre;
R², R³ and R⁴ are identical or different from each other and denote H, hydroxy, alkyloxy, carboxyalkyloxy and esters und amides derived therefrom, hydroxyalkyloxy, aminoalkyloxy, acyloxy, carboxyalkylcarbonyloxy, sulfato, phosphato, halogen, or a modified carbohydrate radical of the formula (II) which is bonded via its anomeric centre, wherein R2 additionally can denote amino or acylamino, and/or wherein two of the radicals R², R³ and R⁴ together can form an epoxy moiety.

Of the conjugates of the formula (I), according to a further preferred embodiment those conjugates are particularly preferred in which
- Sp2: is arylene which is substituted in ortho, meta or para position with K and Sp1 and can additionally carry 1 to 4 further radicals which are identical or different from each other and are selected from the group consisting of H, methyl, methoxy, hydroxy, carboxy, methyloxycarbonyl, cyano, nitro, halogen, sulfonyl oder sulfonamide,
or is a linear or branched alkylene radical,
and the other radicals CT, LI, Sp1 and K are as defined above.

Of the conjugates of the formula (I), according to yet a further preferred embodiment those conjugates are particularly preferred in which
- CT: is camptothecin, which can be linked to the rest of the conjugate via the C20-OH group;
and the other radicals LI, Sp1, Sp2, and K are as defined above.

The compounds of the formula (I) according to the invention can also be present in the form of their salts. In general salts with organic or inorganic bases or acids may be mentioned here.

In particular, the compounds of the formula (I) according to the invention can be employed in the form of their physiologically acceptable salts. Physiologically acceptable salts are understood according to the invention as meaning non-toxic salts which in general are accessible by reaction of the compounds of the formula (I) according to the invention with an inorganic or organic base or acid conventionally used for this purpose. Examples of preferred salts of the compounds of the formula (I) according to the invention are the corresponding alkali metal salt, e.g. lithium, potassium or sodium salt, the corresponding alkaline earth metal salt such as the magnesium or calcium salt, a quaternary ammonium salt such as, for example, the triethylammonium salt, acetate, benzenesulphonate, benzoate, dicarbonate, disulphate, ditartrate, borate, bromide, carbonate, chloride, citrate, dihydrochloride, fumarate, gluconate, glutamate, hexylresorcinate, hydrobromide, hydrochloride, hydroxynaphthoate, iodide, isethionate, lactate, laurate, malate, maleate, mandelate, mesylate, methylbromide, methylnitrate, methylsulphate, nitrate, oleate, oxalate, palmitate, pantothenate, phosphate, diphosphate, polygalacturonate, salicylate, stearate, sulphate, succinate, tartrate, tosylate and valerate and other salts used for medicinal purposes.

The present invention includes both the individual enantiomers or diastereomers and the corresponding racemates, diastereomer mixtures and salts of the compounds according to the invention. In addition, all possible tautomeric forms of the compounds described above are included according to the present invention. Furthermore, the present invention includes both the pure E and Z isomers of the compounds of the formula (I) and their E/Z mixtures in all ratios. The diastereomer mixtures or E/Z mixtures can be separated into the individual isomers by chromatographic processes. The racemates can be resolved into the respective enantiomers either by chromatographic processes on chiral phases or by resolution.

In the context of the present invention, the substituents, if not stated otherwise, in general have the following meaning:
Alkyl in general represents a straight-chain or branched hydrocarbon radical having 1 to 20 carbon atoms. Examples which may be mentioned are methyl, ethyl, propyl, isopropyl, butyl, isobutyl, pentyl, isopentyl, hexyl, isohexyl, heptyl, isoheptyl, octyl and isooctyl, nonyl, decyl, dodeyl, eicosyl.
Alkenyl in general represents a straight-chain or branched hydrocarbon radical having 2 to 20 carbon atoms and one or more, preferably having one or two, double bonds. Examples which may be mentioned are allyl, propenyl, isopropenyl, butenyl, isobutenyl, pentenyl, isopentenyl, hexenyl, isohexenyl, heptenyl, isoheptenyl, octenyl, isooctenyl.
Alkinyl in general represents a straight-chain or branched hydrocarbon radical having 2 to 20 carbon atoms and one or more, preferably having one or two, triple bonds. Examples which may be mentioned are ethinyl, 2-butinyl, 2-pentinyl and 2-hexinyl.
Acyl in general represents straight-chain or branched lower alkyl having 1 to 9 carbon atoms, which is bonded via a carbonyl group. Examples which may be mentioned are: acetyl, ethylcarbonyl, propylcarbonyl, isopropylcarbonyl, butyl-carbonyl and isobutylcarbonyl.
Alkoxy in general represents a straight-chain or branched hydrocarbon radical having 1 to 14 carbon atoms and bonded via an oxygen atom. Examples which may be mentioned are methoxy, ethoxy, propoxy, isopropoxy, butoxy, isobutoxy, pentoxy, isopentoxy, hexoxy, isohexoxy, heptoxy, isoheptoxy, octoxy or isooctoxy, The terms "alkoxy" and "alkyloxy" are used synonymously.
Alkoxyalkyl in general represents an alkyl radical having up to 8 carbon atoms, which is substituted by an alkoxy radical having up to 8 carbon atoms.
Alkoxycarbonyl can be represented, for example, by the formula 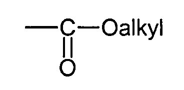 Alkyl here in general represents a straight-chain or branched hydrocarbon radical having 1 to 13 carbon atoms. Examples which may be mentioned are the following alkoxycarbonyl radicals: methoxycarbonyl, ethoxycarbonyl, propoxycarbonyl, isopropoxycarbonyl, butoxycarbonyl or isobutoxycarbonyl.
Cycloalkyl in general represents a cyclic hydrocarbon radical having 3 to 8 carbon atoms. Cyclopropyl, cyclopentyl and cyclohexyl are preferred. Examples which may be mentioned are cyclopentyl, cyclohexyl, cycloheptyl and cyclooctyl.
Cycloalkoxy in the context of the invention represents an alkoxy radical whose hydrocarbon radical is a cycloalkyl radical. The cycloalkyl radical in general has up to 8 carbon atoms. Examples which may be mentioned are: cyclopropyloxy and cyclohexyloxy. The terms "cycloalkoxy" and "cycloalkyloxy" are used synonymously.
Aryl in general represents an aromatic radical having 6 to 10 carbon atoms. Preferred aryl radicals are phenyl, benzyl and naphthyl.
Halogen in the context of the invention represents fluorine, chlorine, bromine and iodine.
Heterocycle in the context of the invention in general represents a saturated, unsaturated or aromatic 3- to 10-membered, for example 5- or 6-membered, heterocycle which can contain up to 3 heteroatoms from the group consisting of S, N and/or O and which, in the case of a nitrogen atom, can also be bonded via this. Examples which may be mentioned are: oxadiazolyl, thiadiazolyl, pyrazolyl, pyridyl, pyrimidinyl, pyridazinyl, pyrazinyl, thienyl, furyl, pyrrolyl, pyrrolidinyl, piperazinyl, tetrahydropyranyl, tetrahydrofuranyl, 1,2,3-triazolyl, thiazolyl, oxazolyl, imidazolyl, morpholinyl or piperidyl. Thiazolyl, furyl, oxazolyl, pyrazolyl, triazolyl, pyridyl, pyrimidinyl, pyridazinyl and tetrahydropyranyl are preferred. The term "heteroaryl" (or "hetaryl") represents an aromatic heterocyclic radical.

The conjugate according to the invention can release its toxophoric radical at its target site and this can thus make possible penetration into the tumour tissue. This is carried out by the specific choice of a unit linking the toxophoric radical to the carbohydrate moiety. The linking unit of the conjugates of the present invention is designed such that it can be cleaved by tumor-associated enzymes such as matrix metalloproteases (MMPs).

A further suitable starting point for promoting the tissue selectivity of the action of the conjugates according to the invention consists in the so-called ADEPT approach. In this, conjugates are cleaved by certain enzymes. These enzymes are introduced into the body coupled to antibodies together with the conjugates according to the invention, the antibodies serving as vehicles specifically addressing tumour tissue. This leads to a selective concentration both of the conjugate and of the enzyme/antibody system in the tumour tissue, whereby the toxophore is released in the tumour tissue with even greater selectivity and can display its action there.

Suitable linking units according to the invention are all linking units which fulfil the abovementioned criteria and can be linked to the carbohydrate moiety.

In the conjugates according to the invention, toxophores used can be all cytotoxic radicals or radicals of a cytostatic or of a cytostatic derivative which are conventionally employed in tumour therapy.

According to a preferred embodiment, conjugates according to the invention which can be employed are compounds of the formula (I) in which a toxophore is linked via a linking unit consisting of 5 to 8 amino acids, preferably 5 to 7 amino acids and particularly preferably 6 amino acids, and of a non-peptide spacer group Sp1 and Sp2, to a carbohydrate moeity.

In the conjugates of the formula (I) according to the invention, the toxophore used can be cytostatic radicals or radicals of a cytostatic or of a cytostatic derivative which are conventionally employed in tumour therapy. Camptothecin or derivatives of camptothecin such as 9-aminocamptothecin are preferred here, which can be linked to the rest of the conjugate via the C20-OH group or via a functional group which is optionally present in the molecule, such as the amino group in the case of 9-aminocamptothecin.

According to this preferred embodiment, the camptothecin unit used as a starting compound can be present in the 20(R) or in the 20(S) configuration or as a mixture of these two stereoisomeric forms. The 20(S) configuration is preferred.

In the conjugates of the formula (I), the linking unit preferably consists of a unit of the formula

-LI-Sp1-Sp2-

wherein the unit LI preferably comprises amino acid residues

-AA1-AA2-AA3-AA4-AA5-AA6-AA7-AA8-

wherein AA1 is bonded to the radical CT, i.e. to the toxophor, and the radical AA8 - or the corresponding radical AA7, AA6 and so on constituting the termination of the linking unit at this end if AA8 is absent - is bonded to the spacer unit Sp1.

According to a preferred embodiment of the present invention, at least 5 of the radicals AA1 to AA8 are present. Particularly preferred is a linking unit wherein 5 to 8 of the radicals AA1 to AA8 are present. Especially preferred is a linking unit wherein 5 to 7 of the radicals AA1 to AA8 are present.According to the most preffered embodiment of the present invention the linking unit comprises 6 of the radicals AA1 to AA8.

The radicals AA1 to AA8, if they are present, each represent an amino acid in the D or L configuration. In this context, they are particularly preferably one of the naturally occurring amino acids glycine, alanine, valine, leucine, isoleucine, serine, threonine, cysteine, methionine, aspartate, glutamate, asparagine, glutamine, arginine, lysine, histidine, tryptophan, phenylalanine, tyrosine or proline. The amino acids used in the process according to the invention can occur in the L or in the D configuration or alternatively as a mixture of D and L form.

The term "amino acids" refers, according to the invention, in particular to the α-amino acids occurring in nature, but moreover also includes their homologues, isomers and derivatives. An example of isomers which can be mentioned is enantiomers. Derivatives can be, for example, amino acids provided with protective groups.

According to the present invention, the amino acids can each be linked to one another and to the toxophore or to the moiety addressing αᵥβ₃ integrin receptors via their α-carboxyl or α-amino functions, but also via functional groups optionally present in side chains, such as, for example, amino functions.

In the case of amino acids having functional groups in the side chains, these functional groups can be either deblocked or protected by conventional protective groups used in peptide chemistry. Protective groups employed for these functional groups of the amino acids can be the protective groups known in peptide chemistry, for example of the urethane, alkyl, acyl, ester or amide type.

Amino protective groups in the context of the invention are the customary amino protective groups used in peptide chemistry. These preferably include: benzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 4-nitrobenzyloxycarbonyl, 2-nitrobenzyloxycarbonyl, 2-nitro-4,5-dimethoxybenzyloxycarbonyl, methoxycarbonyl, ethoxycarbonyl, tert-butoxycarbonyl (Boc), allyloxycarbonyl, vinyloxycarbonyl, 3,4,5-trimethoxybenzyloxycarbonyl, phthaloyl, 2,2,2-trichloro-ethoxycarbonyl, 2,2,2-trichloro-tert-butoxycarbonyl, menthyloxycarbonyl, 4-nitro-phenoxycarbonyl, fluorenyl-9-methoxycarbonyl (Fmoc), formyl, acetyl, propionyl, pivaloyl, 2-chloroacetyl, 2-bromoacetyl, 2,2,2-trifluoroacetyl, 2,2,2-trichloroacetyl, benzoyl, benzyl, 4-chlorobenzoyl, 4-bromobenzoyl, 4-nitrobenzoyl, phthalimido, isovaleroyl or benzyloxymethylene, 4-nitrobenzyl, 2,4-dinitrobenzyl, 4-nitrophenyl or 2-nitrophenylsulphenyl. The Fmoc group and the Boc group are particularly preferred.

The removal of protective groups in appropriate reaction steps can be carried out, for example, by the action of acid or base, hydrogenolytically or reductively in another manner.

According to the present invention, AA1 preferably is a naturally occurring amino acid in the D or L configuration, which is selected from the group consisting of glycine, alanine, valine, leucine, isoleucine, histidine, glutamate, aspartate, serine, lysine, ornithine and phenylalanine. According to the present invention, AA1 most preferably is valine or glycine.

According to the present invention, AA2 preferably is absent or is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine. According to the present invention, AA2 most preferably is a naturally occurring amino acid in the D or L configuration, which is selected from the group consisting of phenylalanine, histidine, asparagine.

According to the present invention, AA3 preferably is absent or is a naturally occurring amino acid in the D or L configuration, which is selected from the group consisting of alanine, valine, phenylalanine, tyrosine, serine, isoleucine, lysine, glutamate, histidine, glycine, arginine, aspartate, tryptophane, proline, ornithine, methionine, S-methyl-cysteine, norvaline and leucine. According to the present invention, AA3 most preferably is a naturally occurring amino acid in the L configuration, which is selected from the group consisting of leucine, norvaline and S-methylcysteine.

According to the present invention, AA4 preferably is absent or is a naturally occurring amino acid in the D or L configuration, which is selected from the group consisting of glycine, alanine, valine, leucine, isoleucine, cysteine and norvaline. According to the present invention, AA4 most preferably is a naturally occurring amino acid in the L configuration, which is selected from the group consisting of glycine, and alanine.

According to the present invention, AA5 preferably is absent or is a naturally occurring amino acid in the D or L configuration, which is selected from the group consisting of glycine, alanine, valine, leucine, isoleucine, histidine, tyrosine, glutamine, asparagine, proline, methionine, phenylalanine and cysteine. According to the present invention, AA5 most preferably is a naturally occurring amino acid in the L-configuration, which is selected from the group consisting of glycine, leucine and glutamine.

According to the present invention, AA6 preferably is absent or is a naturally occurring amino acid in the D or L configuration, which is selected from the group consisting of glycine, alanine, valine, leucine, isoleucine, histidine, glutamine, asparagine, aspartate and proline. According to the present invention, AA6 most preferably is a naturally occurring amino acid in the D or L configuration, which is selected from the group consisting of proline and leucine.

According to the present invention, AA7 preferably is absent or is a naturally occurring amino acid in the D or L configuration, which is selected from the group consisting of glycine, alanine, valine, leucine, isoleucine, histidine, γ-aminobutyric acid, aspartate, glutamate, lysine and proline. According to the present invention, AA7 most preferably is absent or is a naturally occurring amino acid in the D or L configuration, which is selected from the group consisting of glycine, histidine, γ-aminobutyric acid and proline.

According to the present invention, AA8 preferably is absent or is a naturally occurring amino acid in the D or L configuration, which is selected from the group consisting of glycine, alanine, valine, leucine, isoleucine, histidine, lysine, proline and γ-aminobutyric acid. According to the present invention, AA8 most preferably is absent or is a naturally occurring amino acid in the D or L configuration, which is selected from the group consisting of glycine, histidine and γ-aminobutyric acid .

The linking unit LI of the conjugates according to the present invention is designed such that it can selectively be cleaved by tumor-associated enzymes, i.e. enzymes which occur in the tumor tissue and preferably cannot be found in normal tissue or are present in normal tissue only in a significant lower amount as compared with their presence in tumor tissue. As examples of families of tumor-associated enzymes which selectively cleave the linking unit LI of the conjugates according to the present invention may be mentioned matrix metalloproteases (MMPs). According to a particularly preferred embodiment the linking unit LI of the conjugates according to the present invention is designed such that it can selectively be cleaved by certain members of such families of enzymes. Especially preferred the linking unit LI of the conjugates according to the present invention can selectively be cleaved by MMP-2 or MMP-9.

It is preferred according to the invention that the linking unit consists of six to seven amino acids AA1 to AA8 and the spacer unit Sp1 and Sp2, it being possible, in particular, for the unit AA2 to be modified on the side chain by protective groups. However, it is also possible for the linking unit to consist of five or eight amino acids AA1 to AA8 and a spacer unit Sp1 and Sp2. In these cases, the linkage to the toxophore as a rule takes place via the carboxyl function of the amino acid.

According to the present invention, the term "carbohydrate radical K" should mean polyhydroxyaldehydes (aldoses) or polyhydroxyketones (ketoses) as well as high-molecular compounds which can be converted into one of the before-mentioned compounds via hydrolysis. In particular, the term "carbohydrate radical K" should mean monosaccharides, i.e. monomeric polyhydroxyaldehydes or polyhydroxyketones, or oligosaccharides, i.e. dimeric to decameric (disaccharides, trisaccharides etc.) polyhydroxyaldehydes or polyhydroxyketones. As an example for monosaccharides there can be mentioned pentoses and/or hexoses such as ribose, xylose, arabinose, glucose, mannose, galactose, gulose, fructose, sorbose, fucose und rhamnose. As an example for monosaccharides there can be mentioned disaccharides such as saccharose, lactose and maltose. According to the present invention, the carbohydrates can be present in either the D- or L-form. Especially preferred are L-fucose and D-galactose.

According to the present invention, the carbohydrate moieties K can be unsubstituted or regioselectively modified. The term "regioselectively modified" should mean the substitution of one or more hydroxyl groups of the carbohydrate radical by alkyloxy, carboxyalkyloxy or esters or amides derived therefrom such as the corresponding C₁₋₆-alkyl esters oder C₁₋₆-mono- oder dialkyl amides, hydroxyalkyloxy, aminoalkyloxy, acyloxy, carboxyalkylcarbonyloxy, sulfato, phosphato, halogen or optionally amino or acylamino. Furthermore, two of the radicals R², R³ and R⁴ together can form an epoxide moiety. Moreover, one or more hydroxyl groups of the carbohydrate radical can be substituted by one correspondingly modified carbohydrate radical which is bonded via its anomeric centre. One or more hydroxyl groups of the carbohydrate radical can, however, be also replaced by hydrogen, which leads to a conversion of the carbohydrate radical into a so-called desoxy carbohydrate radical.

According to the present invention, especially preferred is the regioselective modification of the carbohydrate radical K via conversion of one or more hydroxyl groups into a C₁₋₆-alkoxy radical such as, for example, methoxy, ethoxy, propoxy or butoxy, into a hydroxyalkyl radical such as, for example, hydroxyethoxy, into a carboxyalkoxy radical or an ester or amide derivative thereof such as, for example, carboxymethoxy, methylcarbonylmethoxy, aminocarbonylmethoxy, methylaminocarbonylmethoxy, ethylaminocarbonylmethoxy, propylaminocarbonylmethoxy, butylaminocarbonylmethoxy, into a carboxyalkyl radical or an ester or amide derivative thereof such as, for example, methylcarbonyloxymethyl, into a carboxy radical or an ester or amide derivative thereof such as, for example, carboxyethyloxycarbonyl, into a halogen radical such as, for example, Cl or Br, or into a sulfate radical.

If one or more hydroxyl groups of the carbohydrate moiety are substitututed by further carbohydrate moeities, independently from the primary carbohydrate moeity these further carbohydrate moeities can be also regioselectively modified or unsubstituted, as mentioned above. According to the present invention, these further carbohydrate moeities are always bonded to the primary carbohydrate moeity via the anomeric centre thereof.

By means of this regioselective modification the compounds of the present invention can be specifically addressed to tumor cells, leading to an increased tissue selectivity and activity.

The carbohydrate moiety K is bonded to the rest of the molecule via a spacer group Sp2. According to the present invention, Sp2 can be an optionally substituted alkylene (also referred to as alkandiyl) or arylene. According to the present invention, Sp2 is preferably a C₂-C₈-alkandiyl radical, i.e. a linear or branched alkandiyl radical having 2 to 8 carbon atoms. Particularly preferred is a linear or branched alkandiyl radical having 2 to 6 carbon atoms. especially preferred is a linear or branched alkandiyl radical having 2 to 4 carbon atoms. As an example there may be mentioned ethylene, propylene, propan-1,2-diyl, propan-2,2-diyl, butan-1,3-diyl, butan-2,4-diyl, pentan-2,4-diyl, 2-methyl-pentan-2,4-diyl. According to the present invention, arylene is an aromatic hydrocarbon radical having 6 to 10 carbon atoms in a cyclic skeleton. As an example there may be mentioned 1,4-phenylene, 1,3-phenylene, 1,2-phenylene or naphthylene. Furthermore, the alkandiyl or arylene spacer moiety can carry one or more substitutens, preferably up to 4 substitutents selected from the group consisting of H, methyl, methoxy, hydroxy, carboxy, methyloxycarbonyl, cyano, nitro, halogen, sulfonyl oder sulfonamide.

The novel conjugates according to Claim 1 can be prepared by linkage of the toxophore to the linking unit and subsequent linkage to the carbohydrate moiety. However, it is also possible to first connect the carbohydrate moiety to the linking unit and then to bind the toxophore to the linking unit.

The combination of the individual units of the conjugates according to the invention can preferably be carried out by means of functional groups which can be reacted with one another and, as a result, can be linked by conventional processes known to the person skilled in the art. For example carboxyl functions can be reacted with amino functions with formation of an amide bond. It is also possible to synthesize the linking unit stepwise on one of the two radicals to be connected, i.e. the toxophore or the carbohydrate moiety, by conventional processes known to the person skilled in the art and then to link the finished linking unit to the radical which is still to be bound.

The present invention in particular relates to a process for the preparation of conjugates according to formula (I), comprising
the reaction of a compound of the formula (III)

K-Sp2-NH₂ (III)

wherein K and Sp2 are as defined in claim 1,
with a carbonic acid derivative such as, for example, phosgene, thiophosgene or a chloroformic acid ester, if appropriate in the presence of a base,
followed by the reaction with a compound of the formula (IV) which has a free primary or secondary amino group

CT-LI (IV)

wherein CT and LI are as defined in claim 1,
and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid.

The synthesis of compounds of the formula (III) is, for example, known from WO 96/31532 (examples 1 to 18), the respective content thereof being expressively incorporated into the present application by reference.

The synthesis of compounds of the formula (IV) is also, for example, known from WO 96/31532 (examples 1 to 18), the respective content thereof being expressively incorporated into the present application by reference.

The term chloroformic acid ester denotes any ester of chloroformic acid such as methyl ester, ethyl ester, or p-nitrophenyl ester. Particularly preferred is the use of p-nitrophenyl chloroformiate.

The above mentioned reaction of the compound of the formula (III) with a carbonic acid derivative can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with ice-cooling and under normal pressure is preferred. According to the present invention, the reaction is preferably carried out in THF at room temperature and under normal pressure. The compounds are used in equimolar amounts. However, the carbonic acid derivative can also be used in slight excess compared to the amount of K-Sp2-NH₂ used. Generally, the reaction is terminated after a few minutes (ca. 5 to 45 minutes).

This reaction is carried out in the presence of a base. Bases which can be employed in the process according to the invention are, for example, triethylamine, ethyl-diisopropylamine, pyridine, N,N-dimethylaminopyridine or other bases conventionally used in steps of this type. Generally, the base is employed in an equimolar amount or in excess (for example two-fold excess).

The intermediate obtained from this reaction step is then reacted with a compound of the formula (IV) in the presence of a base to the compounds of the present invention. Bases which can be employed in the process according to the invention are, for example, triethylamine, ethyl-diisopropylamine, pyridine, N,N-dimethylaminopyridine or other bases conventionally used in steps of this type. The compounds are used in equimolar amounts. However, the compound of the formula (IV) can also be used in slight excess. Generally, the base is emploeyed in an equimolar amount or in excess (for example two-fold excess). This reaction can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with ice-cooling and under normal pressure is preferred. According to the present invention, the reaction is preferably carried out in DMF or THF at room temperature and under normal pressure. Optionally, the reaction can be accelerated or carried out with higher yields by using ultrasonic waves. The reaction time is in the range of from 30 minutes up to several hours, for example 16 hours.

For reacting the compound of the formula (IV) with the intermediate obtained from the reaction of the compound of the formula (III) with a carbonic acid derivative it may be necessary to activate the free carboxy function of the compound of the formula (IV). For the activation of the carboxyl group, the coupling reagents known in peptide chemistry can be used, such as are described, for example, in Jakubke/Jeschkeit: Aminosäuren, Peptide, Proteine [Amino acids, Peptides, Proteins]; Verlag Chemie 1982 or Tetrahedr. Lett. 34, 6705 (1993). Examples mentioned are N-carboxylic acid anhydrides, acid chlorides or mixed anhydrides, adducts with carbodiimides, e.g. N,N'-diethyl-, N,N'-diisopropyl- or N,N'-dicyclohexylcarbodiimide, N-(3-dimethylaminopropyl)N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline or propanephosphonic anhydride or isobutyl chloroformate or benzotriazolyloxy-tris-(dimethylamino)-phosphonium hexafluorophosphate, 1-hydroxybenzotriazole or N-hydroxysuccinimide esters. It is furthermore proposed to employ the acid components in the form of a Leuchs' anhydride.

The compounds obtained according to the process explained above can furthermore be derivatized by removal of protective groups which may be present, further substitution of nitrogen atoms present at preferred positions in the preparation process and/or conversion of the compound obtained into the free acid and/or its physiologically acceptable salts. By way of example, the t-butoxycarbonyl groups conventionally used as protective groups for nitrogen atoms are removed in acidic medium, for example by addition of trifluoroacetic acid. Suitable alkylating agents for the derivatization of nitrogen atoms in this step are reagents conventionally used for this purpose, using which, for example, a substituted or unsubstituted alkyl or cycloalkyl radical, a substituted or unsubstituted aryl radical or a saturated or unsaturated, optionally substituted heterocyclic radical can be bonded to the appropriate nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, Mg(OH)₂ or Ca(OH)₂, as a result of which the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, as a result of which one or more of the nitrogen atoms present are protonated.

The compounds of the formula (IV) serving as starting substances can be prepared by conventional methods. The linkage of the toxophore to amino acid units can be carried out by conventional methods of peptide chemistry (cf., for example, Jakubke/Jeschkeit: Aminosauren, Peptide, Proteine [Amino acids, Peptides, Proteins]; Verlag Chemie 1982, Houben-Weyl, Methoden der Organischen Chemie [Methods of Organic Chemistry], Georg Thieme Verlag Stuttgart, Fourth Edition; Volume 15.1 and 15.2, edited by E.Wünsch) and is also described, for example, in WO 96/31532 and WO 98/51703, whose contents are inserted here by means of reference.

According to a preferred embodiment of the present invention, the toxophore is camptothecin or a camptothecin derivative. The linkage of these toxophores to the linking unit can be carried out via the C20 OH group or other functional groups in the molecule.

The camptothecin unit used as a starting compound can be present in the 20(R) or in the 20(S) configuration or as a mixture of these two stereoisomeric forms. The 20(S) configuration is preferred.

After linkage of the first amino acid to camptothecin, diastereomer mixtures can be formed. Pure diastereomers of the compounds according to the invention can be prepared by the processes indicated above, for example, by separating the diastereomers in a suitable manner after coupling of the first amino acid unit to the camptothecin and subsequent protective group removal.

The conjugates according to the invention can be used as active compound components for the production of medicaments against carcinomatous disorders. For this, they can be converted into the customary formulations such as tablets, coated tablets, aerosols, pills, granules, syrups, emulsions, suspensions and solutions in a known manner using inert, non-toxic, pharmaceutically suitable excipients or solvents. Preferably, the compounds according to the invention are used here in an amount such that their concentration in the total mixture is approximately 0.5 to approximately 90% by weight, the concentration, inter alia, being dependent on the corresponding indication of the medicament.

The abovementioned formulations are produced, for example, by extending the active compounds with solvents and/or excipients having the above properties, where, if appropriate, additionally emulsifiers or dispersants and, in the case of water as the solvent, alternatively an organic solvent, have to be added.

The medicaments according to the invention can be administered in a customary manner.

The present invention is illustrated below with the aid of non-restricting examples and comparison examples.

### Examples

In the examples below, all quantitative data, if not stated otherwise, relate to percentages by weight. The mass determinations were carried out by high-performance liquid chromatography-mass spectrometry (HPLC-MS) using the electron spray ionization (ESI) method or by FAB or MALDI mass spectroscopy.

### List of the abbreviations used

- HPLC: - high-performance liquid chromatography
- RP: - reverse phase
- ACN: - acetonitrile
- DMF: - dimethylformamide
- DCM: - dichloromethane
- THF: - tetrahydrofuran
- DIEA: - diisopropylethylamine (Hünig's base)
- NMP: - N-methylpyrrolidone
- TFA: - trifluoroacetic acid
- Fmoc: - fluorenyl-9-methoxycarbonyl
- RT: - room temperature
- MTBE: - methyl tert-butyl ether
- Boc: - tert-butyloxycarbonyl
- TLC: - thin-layer chromatography
- DMAP: - dimethylaminopyridine
- DMSO: - dimethyl sulphoxide
- Abu: - γ-amino butyric acid

### I. Synthesis of starting materials

### I.1 20-O-Valyl-camptothecin trifluoroacetate

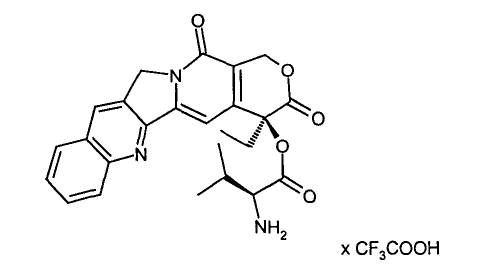

A suspension of 10 g (28.7 mmol) of 20(S)-camptothecin in 500 ml of absolute dichloromethane is treated with stirring with 14 g (2 eq.) of N-(tert-butoxycarbonyl)-valine-N-carboxyanhydride and 1 g of 4-(N,N-dimethylamino)-pyridine. After heating under reflux for 4 days, the mixture is concentrated in vacuo. The residue is stirred with 100 ml of MTBE for 20 min. 200 ml of petroleum ether are then added and the mixture is filtered. 14.9 g of the Boc-protected intermediate compound are obtained, which can contain small amounts of D-valine epimer which, however, can be removed without problems after removal of the protective group.

11.65 g of this Boc-protected intermediate compound are then stirred at 5°C for 1 h in a mixture of 300 ml of dichloromethane and 70 ml of anhydrous trifluoroacetic acid. After concentrating in vacuo to a small volume, the product is precipitated with diethyl ether and thoroughly washed with diethyl ether. The product is again precipitated from dichloromethane/methanol using diethyl ether. If appropriate, the crude product is again taken up in 40 ml of methanol, and the solution is treated with 120 ml of MTBE and cooled to 0°C. The precipitate is filtered off and 9.4 g (80%) of 20-O-(valyl)-camptothecin trifluoroacetate are obtained after drying.
[TLC: acetonitrile/water (20:1); R_{f}= 0.39].

### I.2 20-O-[Histidyl-valyl]-camptothecin bis-trifluoroacetate

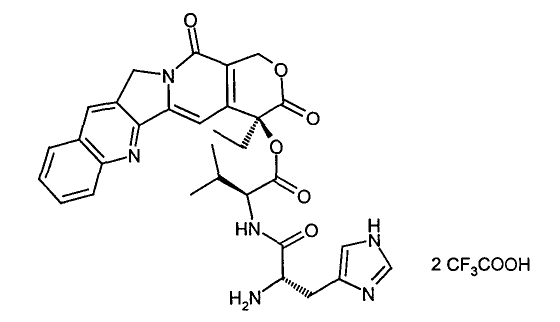

2 g (7.8 mmol) of benzyl N-tert-butoxycarbonyl-histidine are dissolved in 100 ml of DMF and cooled down to 0°C. 1.59 g (1.5 eq) of hydroxybenzotriazole and 1.8 g (1.2 eq) of N-(3-dimethylaminopropyl)-N'-ethylcarbodiimide hydrochloride are added and the mixture is stirrred at 0°C for 30 min. 3.65 g (6.5 mmol) of the compound from Example I.1 and 2.7 ml of Hünig's base are added. The coupling reaction is complete after 16 h. The reaction mixture is poured into 600 ml of MTBE. The precipitating product is collected and it is taken up in dichloromethane. The mixture is extracted twice with water and then the organic phase is dried and concentrated. MTBE is added and the precipitating product is filtered and dried in vacuo. Yield: 4.45g = quantitative; TLC: acetonitrile/water (10:1) R_{f} = 0.33.

4.45 g (6.5 mmol) of this Boc-protected intermediate compound are then stirred at 5°C for 1 hin a mixture of 60 ml of dichloromethane and 30 ml of anhydrous trifluoroacetic acid. After concentrating in vacuo, the product is taken up in dichloromethane/methanol, precipitated with MTBE and thoroughly washed with MTBE. The product is again precipitated from dichloromethane/methanol using MTBE. The precipitate is filtered off and 4.75 g (91%) of the target compound are obtained after drying.
[TLC: acetonitrile/water/glacial acetic acid 5/1/0.2 R_{f}= 0.3].

### I.3 N-tert-butoxycarbonyl-prolyl-leucyl-glycyl-leucin

Commercially available educts N-tert.Butoxycarbonyl-proline hydroxysuccinimide ester (687 mg; 2.2 mmol) and Leucyl-glycyl-leucin (602.8 mg; 2 mmol) are dissolved in 10 ml DMF, 1035 µl Ethyl-diisopropylamine are added and the mixture is sonificated for 18h. Subsequently, the solvent is removed in vacuo and the residue is dissolved in dichloromethane and filtered. The crude product is purified by flash chromatography at silicagel using acetonitrile/water 20:1 as eluent. Relevant fractions are collected and concentrated. The remaining residue is dissolved in dichloromethane and washed with citric acid. The organic layer is dried upon sodium sulfate and the solvent is removed. The product is obtained in a yield of 420mg (42%).
[ESI-MS: m/e = 499 = (M+H)⁺]

### I.4 N-tert-butoxycarbonyl-prolyl-leucyl-glycyl-leucyl-asparagine

This compound is synthesized by coupling of 1.3 with an asparagine derivative or via alternative routes following standard procedures as described in Houben Weyl; Methoden der Organischen Chemie; Vierte Auflage; Band XV Teil 1 und 2; Georg Thieme Verlag Stuttgart 1974, or in Hans-Dieter Jakubke and Hans Jeschkeit: Aminosäuren, Peptide, Proteine; Verlag Chemie, Weinheim 1982.
[TLC: acetonitrile/water/glacial acetic acid 5/1/0.2 R_{f}= 0.68].

### I.5 N-tert-butoxycarbonyl-prolyl-leucyl-glycyl-leucin-histidine

This compound is synthesized following standard procedures.
[TLC: acetonitrile/water/glacial acetic acid 5/1/0.2 R_{f} = 0.28].

### I.6 N-fluorenyl-9-methoxycarbonyl-prolyl-leucyl-glycyl-leucin-asparagine-glycine

This compound is synthesized following standard procedures.
[TLC: acetonitrile/water/glacial acetic acid 5/1/0.2 R_{f}= 0.55].

### II. Preparation of camptothecin petide conjugates

### II.1 20-O-[Prolyl-leucyl-glycyl-leucyl-histidyl-valyl]-camptothecin bis-trifluoroacetate

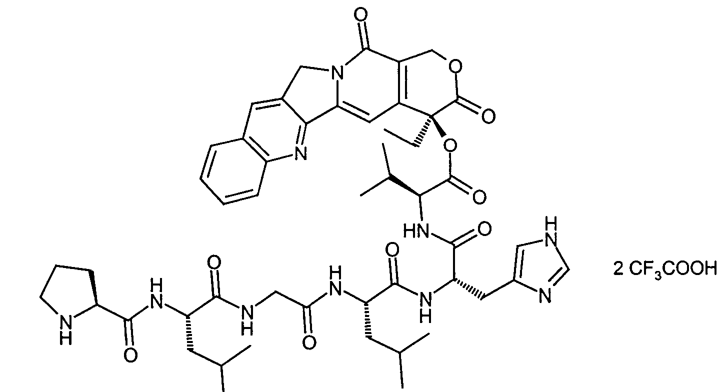

277.4 mg (0.57 mmol) of the compound 1.3 are dissolved in 80 ml of DMF. 116 mg (0.86 mmol) of 1-hydroxy-1H-benzotriazole and 131.7 mg (0.69 mmol) of N-(3-dimethylaminopropyl)-N'-ethylcarbodiimide hydrochloride are added and furthermore 400 mg (0.57 mmol) of the compound from Example I.2 and 222 mg of Hünig's base. The coupling reaction is complete after 2 h. The reation mixture is concentrated and the residue is treated with water. The crude product is purified by flash chromatography at silicagel using dichloromethane/-methanol/ammonia 17% 15/1/0.1 as eluent. Relevant fractions are collected and concentrated. The product is obtained in a yield of 472 mg (77%) [TLC: Acetonitrile/water 10/1 R_{f}= 0.2].

470 mg (0.44 mmol) of this Boc-protected intermediate compound are stirred at 5°C for 1 h in a mixture of 50 ml of dichloromethane and 10 ml of anhydrous trifluoroacetic acid. After concentrating in vacuo, the product is taken up in dichloromethane/methanol, precipitated with diethyl ether and filtered off. The residue is again precipitated from dichloromethane/methanol using diethyl ether. The precipitate is filtered off and, after drying, 432 mg (91%) of the target compound are obtained.
[TLC: acetonitrile/water/glacial acetic acid 5/1/0.2 R_{f}= 0.15].

In analogy to I.1, further camptothecin amino acid conjugates were prepared by reaction of camptothecin with partially protected amino derivatives. Subsequently, the peptide chain is elongated either by attachment of single amino acid derivatives and subsequent deprotection of the amino terminus or by fragment condensation as exemplified in II.1 or in a combination of both. If appropriate, protective groups are removed. Coupling-, protection- and deprotection-steps are performed according to known methods as reported in: Houben Weyl; Methoden der Organischen Chemie; Vierte Auflage; Band XV Teil 1 und 2; Georg Thieme Verlag Stuttgart 1974; Hans-Dieter Jakubke and Hans Jeschkeit: Aminosäuren, Peptide, Proteine; Verlag Chemie, Weinheim 1982. The conjugates prepared according to this method are shown below:

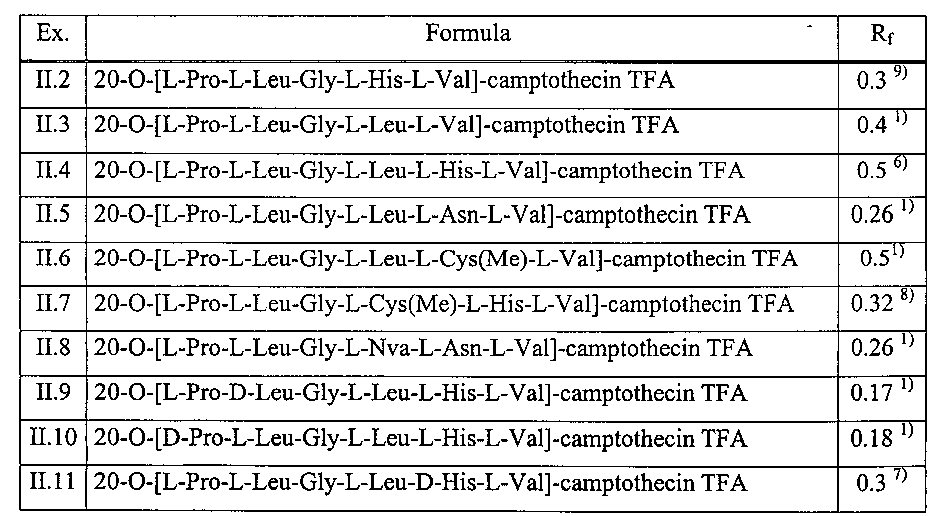

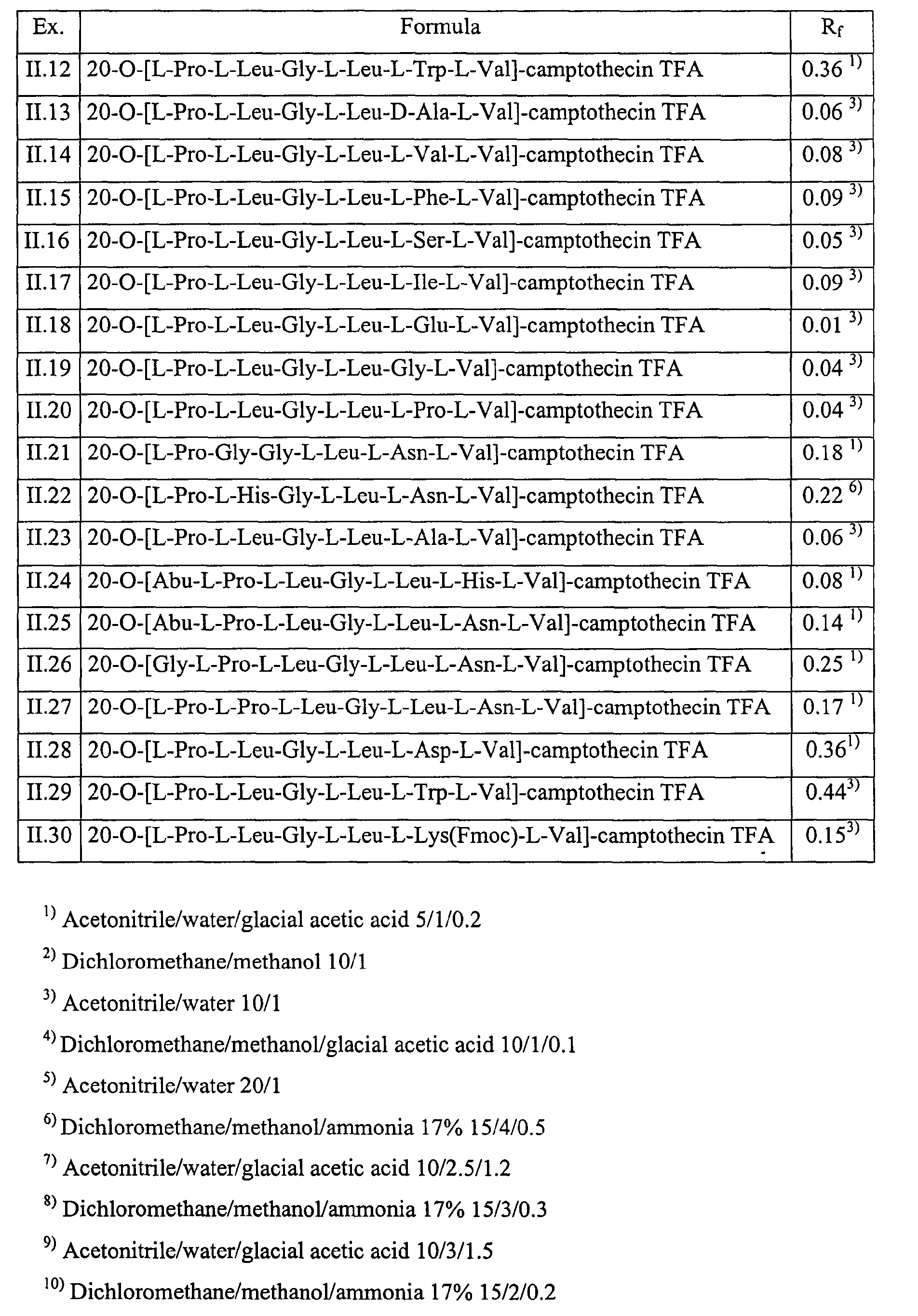

### III. Preparation of carbohydrate ligands

The synthesis of the carbohydrate ligands is disclosed in WO 96/31532 (example 1), the content thereof being explicitely incorported into the present application by reference.

### IV Preparation of glycoconjugates of cytotoxic agents

### General procedure A (thiourea linkage)

0.89 mmol of a carbohydrate derivative from series III are dissolved in 2400 ml of dioxane/water (1:1) and treated with 9.6 ml (1.4 eq.) of thiophosgene. After stirring at room temperature for 15 min 91 ml (6 eq.) of Hünig's base are added and the mixture is stirred for another 10 min and then concentrated. The residue is taken up in dichloromethane and precipitated using diethylether, MTBE or a mixture of MTBE and petroleum ether. The corresponding isothiocyanates are obtained in yields exceeding 80% and they are reacted in the next step without further purification.

0.04 mmol of the isothiocyanate is dissolved in 10 ml of DMF and then treated with 0.02 mmol of one of the peptide conjugates in series II (II1-IIn) in the presence of 12 µl of Hünig's base. After stirring at room temperature overnight, the mixture is concentrated and the residue the residue is precipitated from methanol/dichloromethane using diethylether.

If neccessary, further purification is done by flash chromatography at silica gel. Appropriate eluent systems are:
Dichloromethane/methanol/ammonia 17% 15/3/0.3
Dichloromethane/methanol/ammonia 17% 16/2/0.2

The relevant fractions are collected, concentrated and the target products are isolated by precipitation from methanol/dichloromethane using diethylether.

### General procedure B (urea linkage)

0.06 mmol 4-Nitrophenyl chloroformic acid ester are dissolved in 2 ml THF and 13 µl Hünig's base are added. Subsequently, 0.04 mmol of one of the carbohydrate derivatives from series III dissolved in 2 ml THF are added and the mixture is stirred at room temperature for 10 min.

Subsequently, 0.033 mmol of a peptide conjugate from series II (II.1 - II.n) in 2 ml DMF and 10 µl Hünig's base are added and the mixture is stirred overnight at room temperature. The solvent is removed and the residue is purified by flash chromatography at silica gel. Appropriate eluent mixtures are:
Dichloromethane/methanol/ammonia 17% 15/1/0.1
Dichloromethane/methanol/ammonia 17% 16/3/0.3

The relevant fractions are collected, concentrated and the target products are isolated by precipitation from methanol/dichloromethane using ether. Alternatively, the glycoconjugates may also be isolated by lyophilization of a solution in dioxane/water 1:1.

Subsequently, if the target compounds contain basic or acidic functional groups, they may be transferred in the corresponding salts such as hydrochlorides or sodium salts using aequeous hydrochloride solution and sodiumhydroxide, respectively.

### Example 1

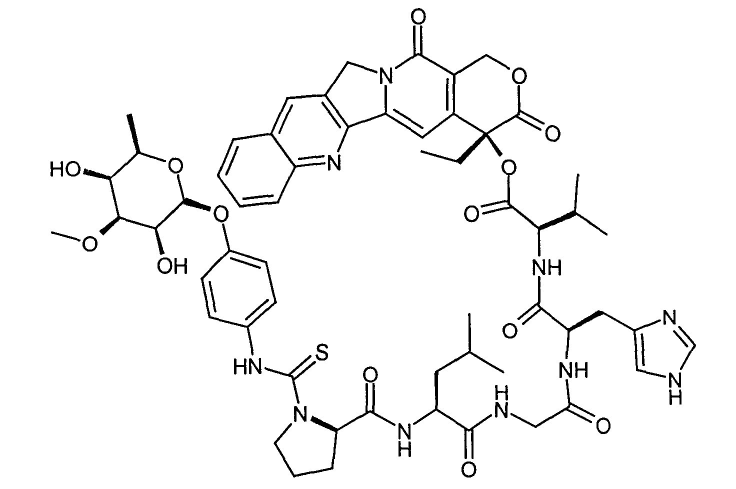

- Educts:: II.2, and the carbohydrate ligand from WO 96/31532, ex. 1.2
- Procedure:: A
- Yield:: 78%
R_{f}= 0.3 (Acetonitrile/water/glacial acetic acid 5/1/0.2)

### Example 2

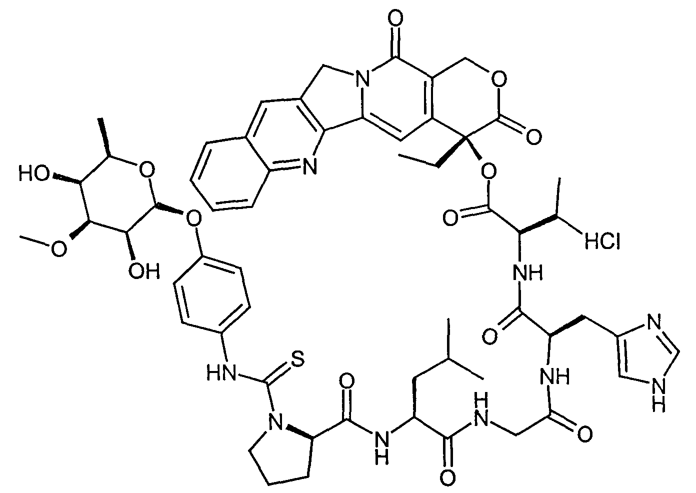

Transformation of example 1 into the corresponding hydrochloride using 1 eq. of 0.1 N HCl and subsequent lyophilization.
Yield: 97%
R_{f}= 0.3 (Acetonitrile/water/glacial acetic acid 5/1/0.2)
[ESI-MS: m/e = 1163 = (M+H)⁺]

### Example 3

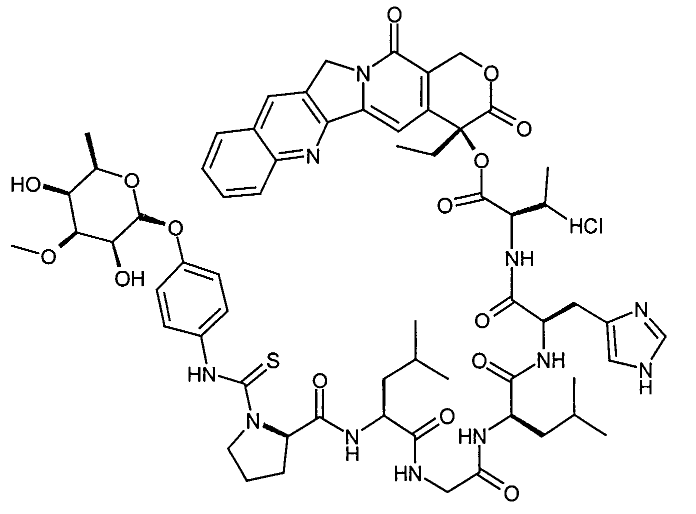

- Educts:: II.4, and the carbohydrate ligand from WO 96/31532, ex. 1.2
- Procedure:: A and subsequent transformation into the corresponding hydrochloride using 1 eq. of 0.1 N HCl. The product is isolated by lyophilization of the aequeous solution.
- Yield:: 66%
R_{f}= 0.16 (Acetonitrile/water 10/1)
[ESI-MS: m/e = 1276 = (M+H)⁺]

### Example 4

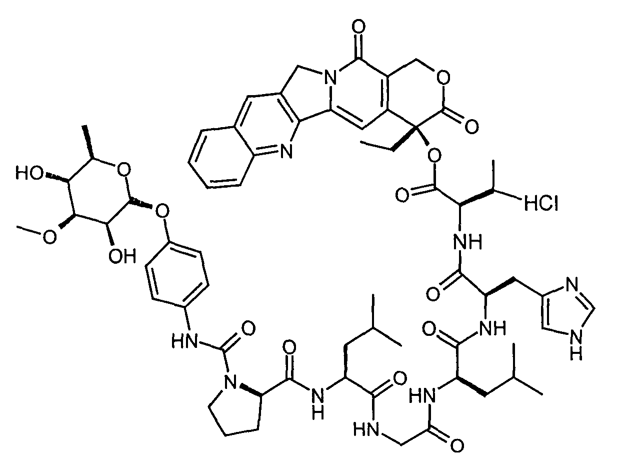

- Educts:: II.4, and the carbohydrate ligand from WO 96/31532, ex. 1.2
- Procedure:: B and subsequent transformation into the corresponding hydrochloride using 1 eq. of 0.1 N HCl. The product is isolated by lyophilization of the aequeous solution.
- Yield:: 49%
R_{f} = 0.15 (Dichloromethane/methanol/ammonia 17% 15/2/0.2)
[ESI-MS: m/e = 1260 = (M+H)⁺]

### Example 5

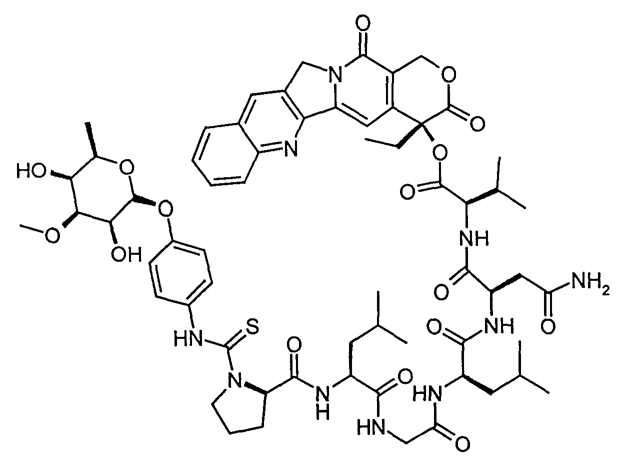

- Educts:: II.5, and the carbohydrate ligand from WO 96/31532, ex. 1.2
- Procedure:: A
- Yield:: 78%
R_{f}= 0.34 (Acetonitrile/water 10/1)
[ESI-MS: m/e = 1253 = (M+H)⁺]

### Example 6

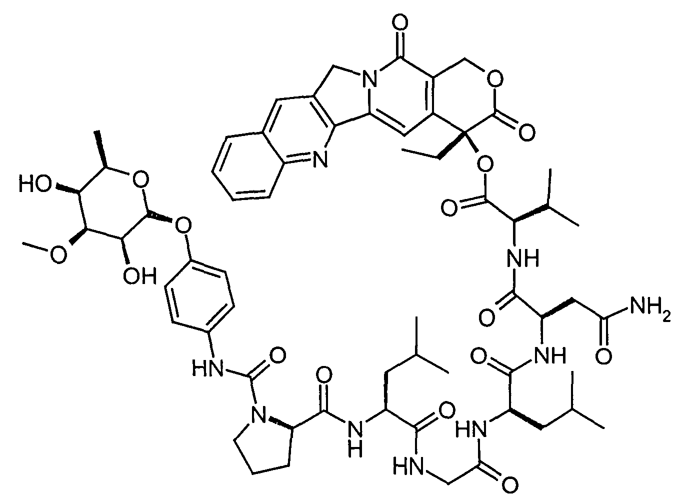

- Educts:: II.5, and the carbohydrate ligand from WO 96/31532, ex. 1.2
- Procedure:: B
- Yield:: 60%
R_{f}= 0.36 (Dichloromethane/methanol/glacial acetic acid 10/1/0.1)
[ESI-MS: m/e = 1237 = (M+H)⁺]

### Example 7

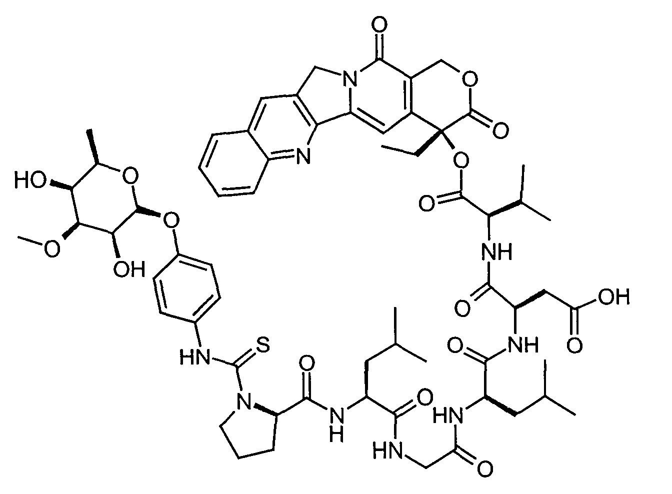

- Educts:: II.28, and the carbohydrate ligand from WO 96/31532, ex. 1.2
- Procedure:: A
- Yield:: 76%
R_{f} = 0.44 (Dichloromethane/methanol/glacial acetic acid 10/1/0.1)
[ESI-MS: m/e = 1254 = (M+H)⁺]

### Example 8

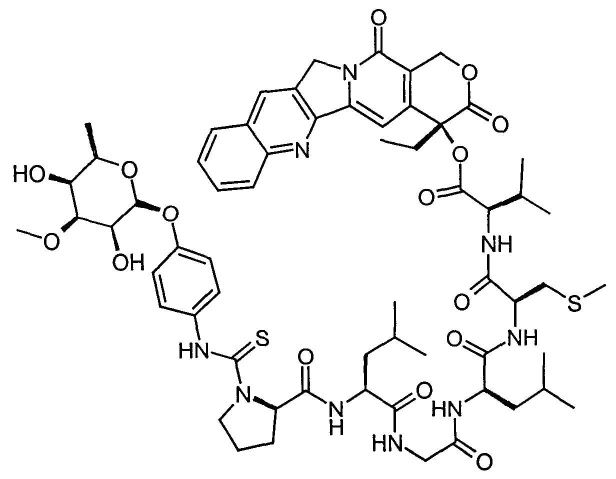

- Educts:: II.6, and the carbohydrate ligand from WO 96/31532, ex. 1.2
- Procedure:: A
- Yield:: 80%
R_{f}= 0.28 (Acetonitrile/water 20/1)
[ESI-MS: m/e = 1256 = (M+H)⁺]

### Example 9

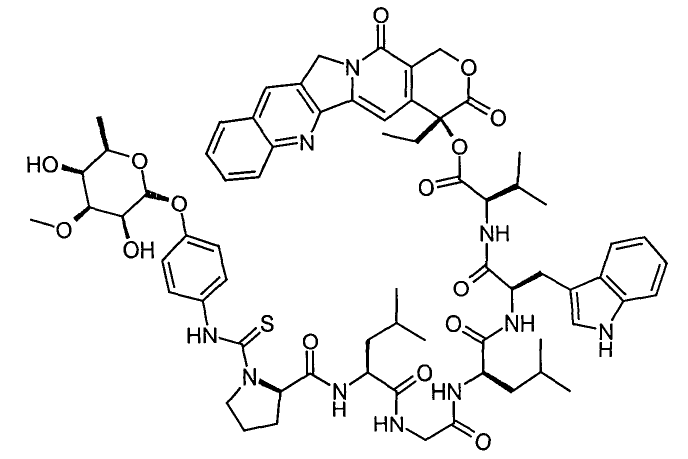

- Educts:: II.29, and the carbohydrate ligand from WO 96/31532, ex. 1.2
- Procedure:: A
- Yield:: 94%
R_{f}= 0.55 (Dichloromethane/methanol/glacial acetic acid 10/1/0.1)
[ESI-MS: m/e = 1325 = (M+H)⁺]

### Example 10

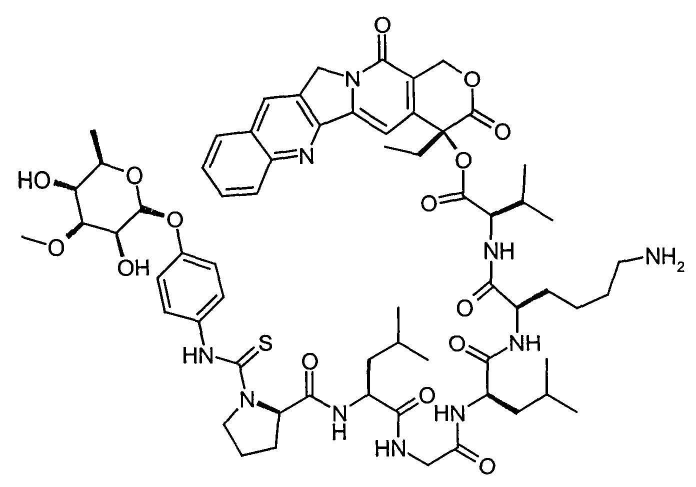

- Educts:: II.30, and the carbohydrate ligand from WO 96/31532, ex. 1.2
- Procedure:: A
- Yield (coupling step):: 91%
R_{f}= 0.3 (Dichloromethane/methanol/ammonia 17% 15/2/0.2)
Subsequent cleavage of the Fmoc group with piperidine in DMF:
- Yield (deprotection step):: 12%
R_{f} = 0.23 (Dichloromethane/methanol/ammonia 17% 15/4/0.5)
[ESI-MS: m/e = 1267 = (M+H)⁺]

### Example 11

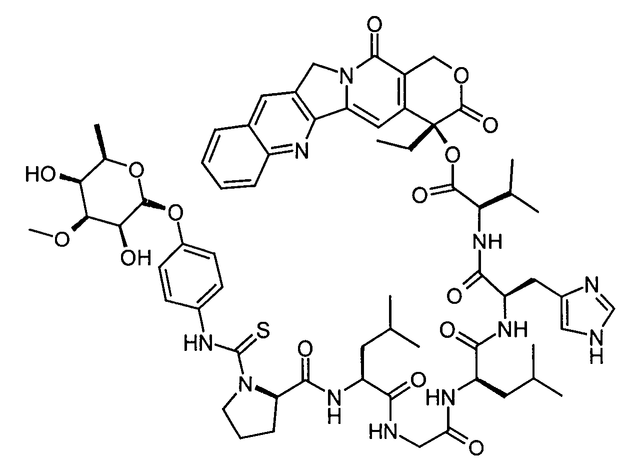

- Educts:: II.11, and the carbohydrate ligand from WO 96/31532, ex. 1.2
- Procedure:: A
- Yield:: 59%
R_{f}= 0.4 (Acetonitrile/water/glacial acetic acid 5/1/0.2)
[ESI-MS: m/e = 1276 = (M+H)⁺]

### Example 12

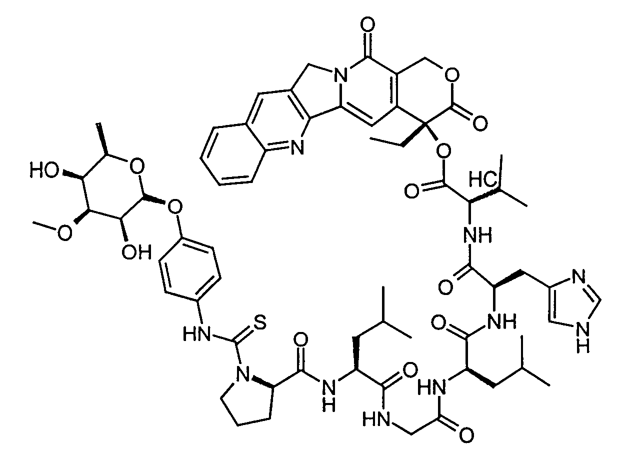

Educts: II.9, and the carbohydrate ligand from WO 96/31532, ex. 1.2
Procedure: A; subsequent formation of hydrochloride
Yield: 72%
R_{f}= 0.53 (Acetonitrile/water/glacial acetic acid 5/1/0.2)
[ESI-MS: m/e = 1276 = (M+H)⁺]

### Example 13

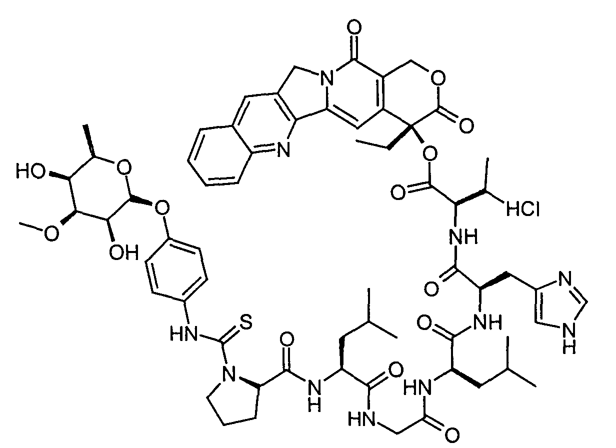

- Educts:: II.10, and the carbohydrate ligand from WO 96/31532, ex. 1.2
- Procedure:: A; subsequent formation of hydrochloride
- Yield:: 17%
R_{f}= 0.43 (Acetonitrile/water/glacial acetic acid 5/1/0.2)
[ESI-MS: m/e = 1276 = (M+H)⁺]

### Example 14

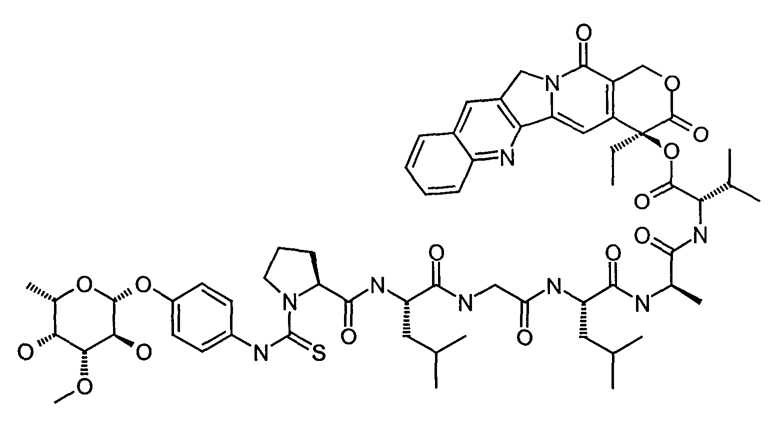

- Educts:: II.13 and the carbohydrate ligand from WO 96/31532, ex. 1.2
- Procedure:: A
- Yield:: 33%
R_{f} = 0.58 (acetonitrile/water 10/1)
[ESI-MS: m/e = 1210.2 = (M+H)⁺]

### Example 15

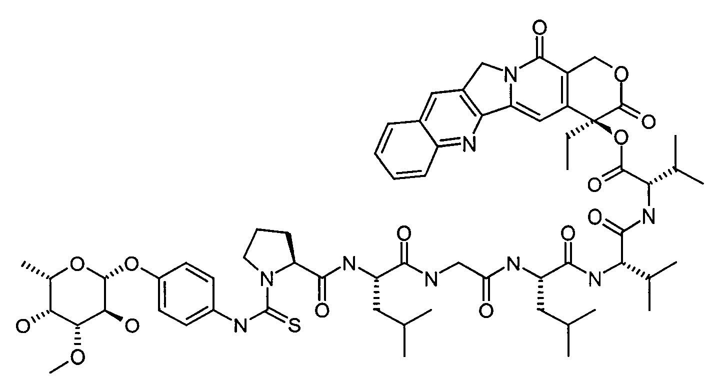

- Educts:: II.14 and the carbohydrate ligand from WO 96/31532, ex. 1.2
- Procedure:: A
- Yield:: 35%
R_{f} = 0.6 (acetonitrile/water 10/1)
[ESI-MS: m/e = 1238.7 = (M+H)⁺]

### Example 16

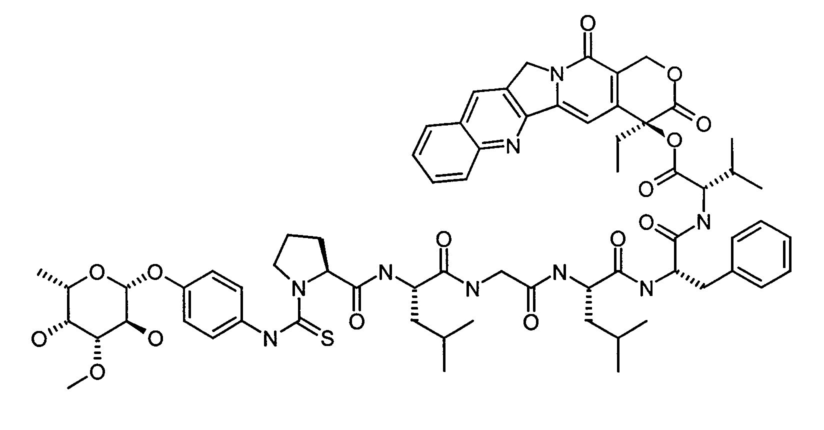

- Educts:: II.15 and the carbohydrate ligand from WO 96/31532, ex. 1.2
- Procedure:: A
- Yield:: 59% R_{f} = 0.66 (acetonitrile/water 10/1)
[ESI-MS: m/e = 1286.9 = (M+H)⁺]

### Example 17

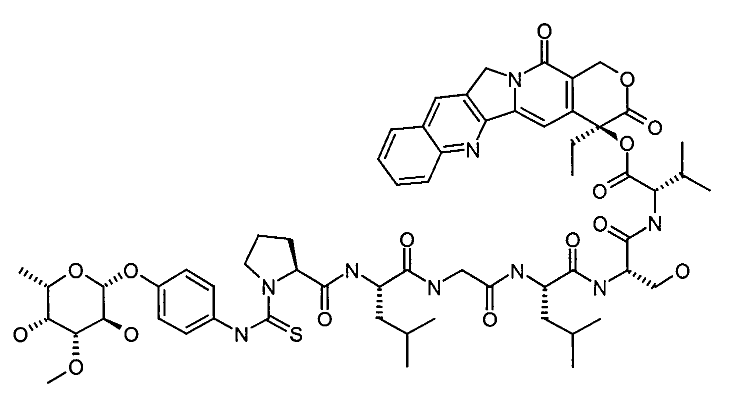

- Educts:: II.16 and the carbohydrate ligand from WO 96/31532, ex. 1.2
- Procedure:: A
- Yield:: 44% R_{f} =0.54 (acetonitrile/water 10/1)
[ESI-MS: m/e = 1226.7 = (M+H)⁺]

### Example 18

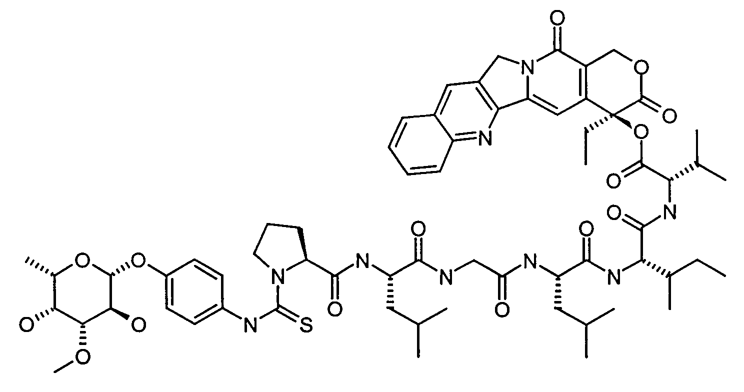

- Educts:: II.17 and the carbohydrate ligand from WO 96/31532, ex. 1.2
- Procedure:: A
- Yield:: 53% R_{f} =0.65 (acetonitrile/water 10/1)
[ESI-MS: m/e = 1252.7 = (M+H)⁺]

### Example 19

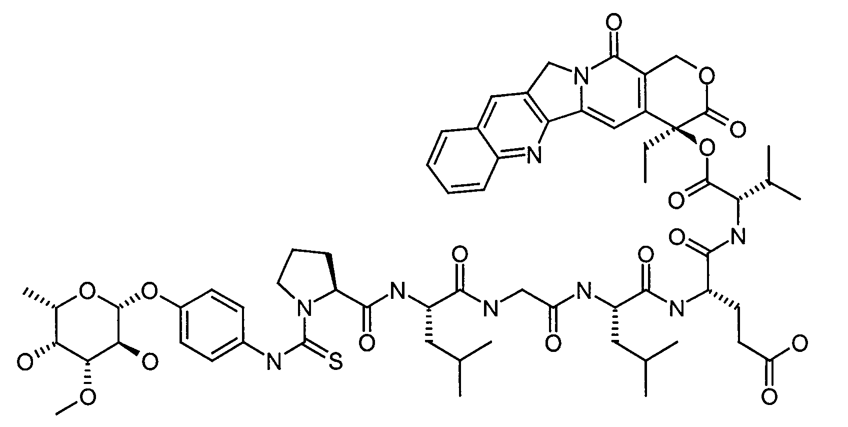

- Educts:: II.18 and the carbohydrate ligand from WO 96/31532, ex. 1.2
- Procedure:: A
- Yield:: 65% R_{f}= 0.27 (acetonitrile/water 10/1)
[ESI-MS: m/e = 1268.6 = (M+H)⁺]

### Example 20

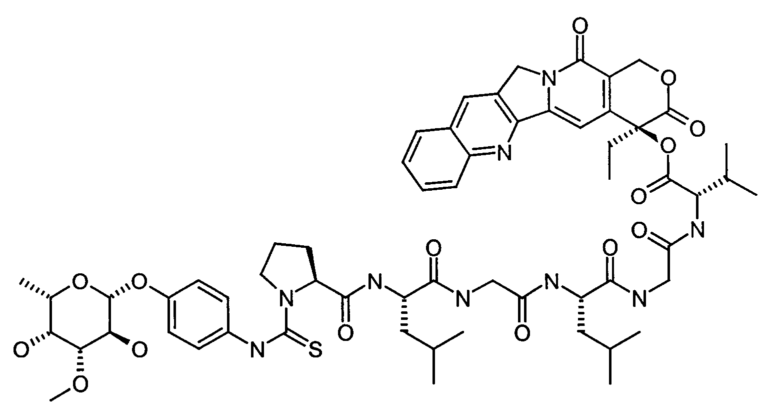

- Educts:: II.19 and the carbohydrate ligand from WO 96/31532, ex. 1.2
- Procedure:: A
- Yield:: 60% R_{f} = 0.59 (acetonitrile/water 10/1)
[ESI-MS: m/e = 1196.7 = (M+H)⁺]

### Example 21

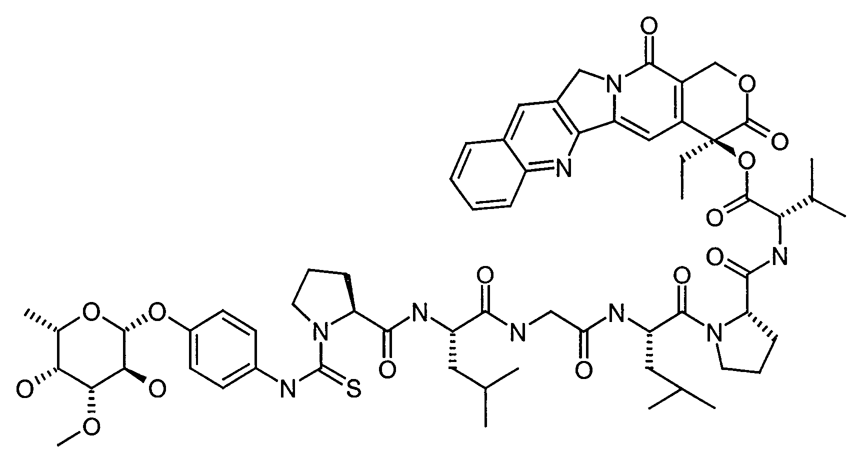

- Educts:: II.20 and the carbohydrate ligand from WO 96/31532, ex. 1.2
- Procedure:: A
- Yield:: 54% R_{f}= 0.52 (acetonitrile/water 10/1)
[ESI-MS: m/e = 1236.7 = (M+H)⁺]

### Example 22

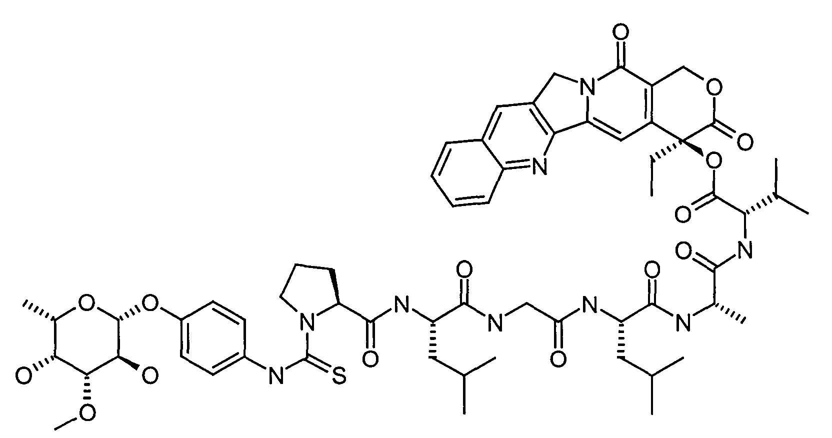

- Educts:: II.23 and the carbohydrate ligand from WO 96/31532, ex. 1.2
- Procedure:: A
- Yield:: 33% R_{f}= 0.59 (acetonitrile/water 10/1)
[ESI-MS: m/e = 1210.2 = (M+H)⁺]

### Example 23

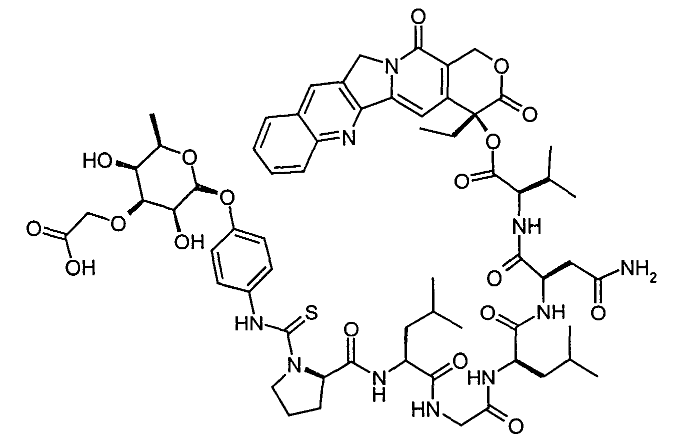

- Educts:: II.5 and the carbohydrate ligand from WO 96/31532, ex. 1.10
- Procedure:: A
- Yield:: 75% R_{f} = 0.1 (acetonitrile/water 10/1)
[ESI-MS: m/e = 1297 = (M+H)⁺]

### Example 24

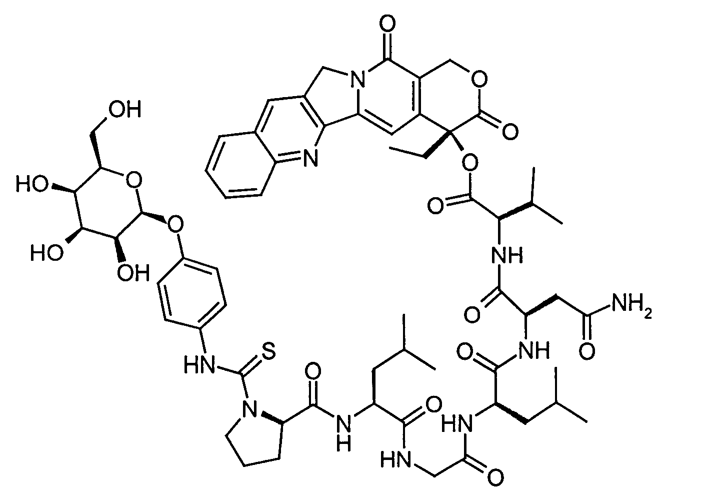

- Educts:: II.5 and the carbohydrate ligand from WO 96/31532, ex. 1.23
- Procedure: Yield:: 57% R_{f} = 0.2 (acetonitrile/water 10/1)
[ESI-MS: m/e = 1255 = (M+H)⁺]

### Example 25

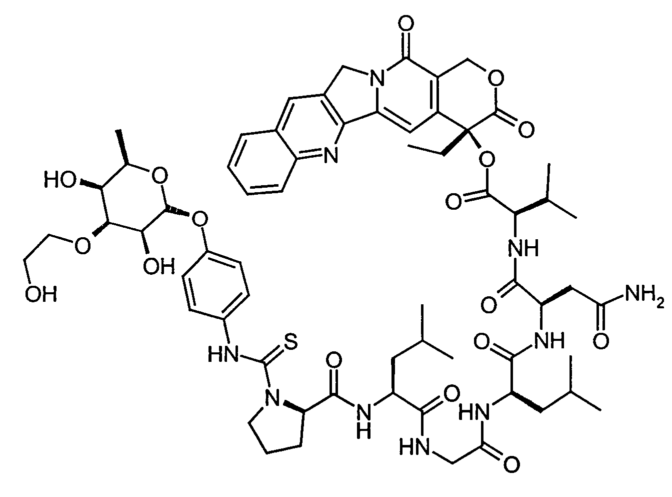

- Educts:: II.5 and the carbohydrate ligand from WO 96/31532, ex. 1.12
- Procedure:: A
- Yield:: 68% R_{f} = 0.29 (acetonitrile/water/glacial acetic acid 10/1/0.1)
[ESI-MS: m/e = 1283 = (M+H)⁺]

### Example 26

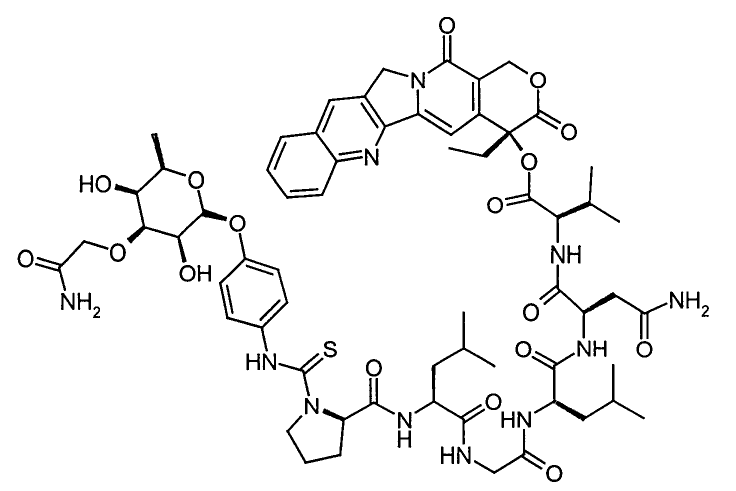

- Educts:: II.5 and the carbohydrate ligand from WO 96/31532, ex. 1.18
- Procedure:: A
- Yield:: 69% R_{f}= 0.28 (acetonitrile/water/glacial acetic acid 10/1/0.1)
[ESI-MS: m/e = 1296 = (M+H)⁺]

### Example 27

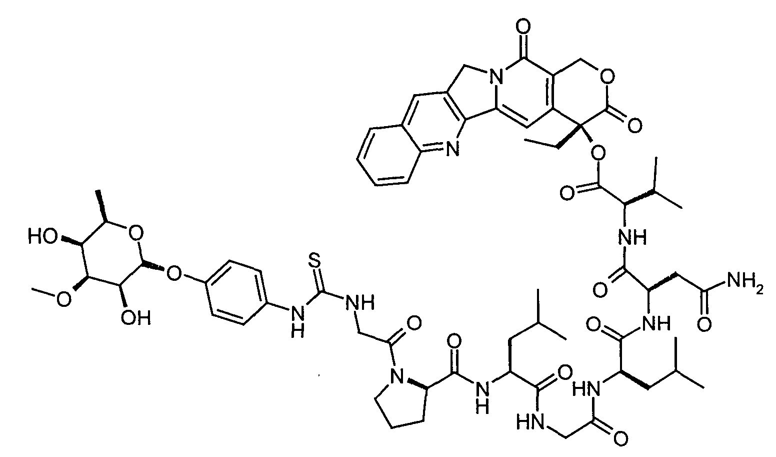

- Educts:: II.26, and the carbohydrate ligand from WO 96/31532, ex. 1.2
- Procedure:: A
Yield:
R_{f} = (Acetonitrile/water 10/1)
[ESI-MS: m/e = = (M+H)⁺]

### Biological Tests

### A: Growth inhibition test for the determination of the cytotoxic properties on various tumour cell lines:

The human large intestine cell lines SW 480 and HT29 (ATCC No. CCL 228 and HTB38 and the mouse melanoma cell line B16F10 (CRL 6475) were grown to confluence in Roux dishes in RPMI 1640 medium with addition of 10% FCS. They were then trypsinized and taken up in RPMI plus 10% FCS to a cell count of 50,000 cells or, for B16F10, 20,000 cells per ml. 100 µl of cell suspension/well were added to a 96 microwell plate and incubated at 37°C for 1 day in a CO₂ incubator. A further 100 µl of RPMI medium and 1 µl of DMSO were then added with the test substances. The growth was checked after day 6. For this, 25 µl of MTT solution (3-(4,5-dimethylthiazol-2-yl)-2,5-diphenyltetrazolium bromide) was added to each well at a starting concentration of 5 mg/ml of H₂O. The plate was incubated at 37°C for 5 hours in a CO₂ incubator. The medium was then aspirated and 100 µl of i-propanol/well were added. After shaking with 100 µl of H₂O for 30 min, the extinction was measured at 595 nm using a Multiplate Reader (BIO-RAD 3550-UV).

The cytostatic action is indicated in Table 1 as an IC₅₀ value, in each case for the individual cell lines.

**Table 1:**

| IC₅₀ values of the cytotoxic action on tumour cell lines [nM] | | | |
|---|---|---|---|
| Example | SW480 | IC50 [nM] HT29 | B16F10 |
| 1 | 150 | 70 | 150 |
| 2 | 150 | 70 | 150 |
| 3 | 100 | 50 | 150 |
| 4 | 50 | 40 | 150 |
| 5 | 80 | 60 | 300 |
| 6 | 60 | 50 | 300 |
| 7 | 200 | 100 | 600 |
| 8 | 50 | 40 | 150 |
| 9 | 400 | 300 | 1000 |
| 10 | 80 | 70 | 150 |
| 11 | 200 | 80 | 300 |
| 12 | 150 | 100 | 300 |
| 13 | 100 | 70 | 200 |
| 14 | 700 | 900 | 2000 |
| 15 | 80 | 100 | 400 |
| 16 | 60 | 80 | 250 |
| 17 | 40 | 70 | 200 |
| 18 | 100 | 200 | 600 |
| 19 | 200 | 400 | 1500 |
| 20 | 120 | 250 | 800 |
| 21 | 100 | 250 | 800 |
| 22 | 40 | 70 | 250 |
| 23 | 200 | 120 | 800 |
| 24 | 200 | 100 | 500 |
| 25 | 180 | 150 | 800 |
| 26 | 150 | 90 | 450 |
| 27 | 25 | 15 | 50 |

### B. In-vivo inhibition or tumour growth using a nude mouse model

### Material

In all in-vivo experiments for investigating the inhibition of tumour growth, athymic nude mice (NMRI nu/nu strain) were used. The tumour was developed by serial passage in nude mice. The human origin of the tumour was confirmed by isoenzymatic and immunohistochemical methods.

### Experimental set-up

The tumour was implanted subcutaneously in both flanks of nu/nu nude mice 6 to 8 weeks old. The treatment was started, depending on the doubling time, as soon as the tumours had reached a diameter of 5 - 7 mm. The mice were assigned to the treatment group or the control group (5 mice per group having 8 - 10 assessable tumours) by randomization. The individual tumours of the control group all grew progressively.

The size of the tumours was measured in two dimensions by means of a slide gauge. The tumour volume, which correlated well with the cell count, was then used for all assessments. The volume was calculated according to the formula "length x breadth x breadth / 2" ([a x b²] / 2, a and b represent two diameters arranged at right angles).

The values of the relative tumour volume (RTV) were calculated for each individual tumour by dividing the tumour size on day X with the tumour size on day 0 (at the time of randomization). The average values of the RTV were then used for the further assessment.

The inhibition of the increase of the tumour volume (tumour volume of the test group/control group, T/C, in per cent) was the final measured value.

### Treatment

The compounds can be administered with a daily or an intermittent therapy schedule through a couple of days either by intraperitoneal, intravenious, oral or subcutaneous route.

In a subcutaneously growing melanoma xenograft model (MEXF 989) several compounds effected inhibitions of the tumor growth (eg. compound of example 2, and 5). The compounds are dissolved in water or in PEG400/water 2:1 and are administered intravenously or intraperitoneally from day 1-3 and day 15-17. The optimal calculated T/C values are given in table 2.

**Table 2**

| Example | dose | lethality | optimal T/C in % |
|---|---|---|---|
| 3 | 8 mg/kg/day | 1/5 | 19.1 |
| 5 | 6 mg/kg/day | 1/5 | 19.1 |

### C. CSF-induced proliferation of hemopoietic stem cells

Bone marrow cells are flushed out of the femur of mice. 10⁵ cells are incubated in McCoy 5A medium (0.3% agar) together with recombinant murine GM-CSF (Genzyme; parent cell colony formation) and the substances (10⁻⁴ to 100 µg/ml) at 37°C and 7% CO₂. 7 days later, the colonies (<50 cells) and clusters (17-50 cells) are counted.

A series of compounds exhibits a drastically reduced toxicity against stem cells in vitro compared to camptothecin (cf. Table 3).

**Table 3:**

| IC₅₀ values of inhibition of colony counts of hemopoietic stem cells [ng/ml] | |
|---|---|
| Example | IC₅₀[ng/ml] |
| 3 | 40 |
| 12 | 30 |
| Camptothecin | 0.25 |

### D. Cleavage of conjugates by MMP-2 in buffer

2.5 µl MMP-2 (Calbiochem) with a specific activity of 60µU/µl are incubated with 10nM of the conjugates exemplified in the example series 1-25 in 1 ml of a medium consisting of 50 mM Tris-HCl pH 7.5, 0.2 M NaCl, 10 mM CaCL₂*2H₂O and 0,05% Brij 35. The enzyme mediated cleavage of the conjugates is detected by HPLC analysis using an RP18 (5µM) column with 70% HClO₄ /water (0.4% v/v) as eluent A and acetonitrile as eluent B (UV detection at 356nM). The efficiency of the cleavage is assessed by comparing the peak areas of the intact conjugate (starting material) and the cleavage product (tripeptide conjugate of camptothecin) after 6 h and 24 h incubation (cf. Table 4).

**Table 4:**

| Ratio of peak areas of cleavage product/starting material after 6 h incubation with MMP-2 | |
|---|---|
| Example | MMP-2 mediated cleavage Peak area product/educt x100 |
| 3 | 609 |
| 4 | 700 |
| 5 | 166 |
| 6 | 80 |
| 7 | 9 |
| 9 | 64 |

## Claims

1. Conjugate, **characterized by** the formula (I)
CT - LI - Sp1- Sp2 - K (I)
in which
CT denotes a cytotoxic radical or a radical of a cytostatic or of a cytostatic derivative, which can additionally carry a hydroxyl, carboxyl or amino group,
LI is a linker group comprising 5 to 8 amino acid residues in the D or L configuration, which can each optionally carry protective groups,
Sp1 is absent or a carbonyl or a thiocarbonyl radical,
Sp2 is an optionally substituted arylene or alkylene radical,
K is an unsubstituted or regioselectively modified carbohydrate radical;
and their physiologically acceptable salts, hydrates and stereoisomers.

2. Conjugate according to Claim 1, **characterized in that**
LI is a linker group having the formula
-AA1-AA2-AA3-AA4-AA5-AA6-AA7-AA8-
wherein at least 5 of the radicals AA1 to AA8 are present, AA1 is bonded to the radical CT and
AA1 is a naturally occurring amino acid in the D or L configuration, which is selected from the group consisting of glycine, alanine, valine, leucine, isoleucine, histidine, glutamate, aspartate, serine, lysine, ornithine and phenylalanine;
AA2 is absent or is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine, and can optionally carry protective groups,
AA3 is absent or is a naturally occurring amino acid in the D or L configuration, which is selected from the group consisting of alanine, valine, phenylalanine, tyrosine, serine, isoleucine, lysine, glutamate, histidine, glycine, arginine, aspartate, tryptophane, proline, ornithine, methionine, S-methyl-cysteine, norvaline and leucine, and can optionally carry protective groups,
AA4 is absent or is a naturally occurring amino acid in the D or L configuration, which is selected from the group consisting of glycine, alanine, valine, leucine, isoleucine, cysteine and norvaline;
AA5 is absent or is a naturally occurring amino acid in the D or L configuration, which is selected from the group consisting of glycine, alanine, valine, leucine, isoleucine, histidine, tyrosine, glutamine, asparagine, proline, methionine, phenylalanine and cysteine;
AA6 is absent or is a naturally occurring amino acid in the D or L configuration, which is selected from the group consisting of glycine, alanine, valine, leucine, isoleucine, histidine, glutamine, asparagine, aspartate and proline;
AA7 is absent or is a naturally occurring amino acid in the D or L configuration, which is selected from the group consisting of glycine, alanine, valine, leucine, isoleucine, histidine, γ-aminobutyric acid, aspartate, glutamate, lysine and proline;
AA8 is absent or is a naturally occurring amino acid in the D or L configuration, which is selected from the group consisting of glycine, alanine, valine, leucine, isoleucine, histidine, lysine, proline and γ-aminobutyric acid;
and the other radicals CT, Sp1, Sp2 and K are as defined in claim 1.

3. Conjugate according to claim 2, **characterized in that**
LI is a linker group having the formula
-AA1-AA2-AA3-AA4-AA5-AA6-AA7-AA8-
wherein 5 to 7 of the radicals AA1 to AA8 are present, AA1 is bonded to the radical CT and
AA1 is valine, glycine, leucine, histidine;
AA2 is absent or is a naturally occurring amino acid in the D or L configuration, which is selected from the group consisting of alanine, phenylalanine, serine, isoleucine, glutamate, asparagine, glutamine, histidine, glycine, aspartate, tryptophane, proline, and leucine, and can optionally carry protective groups,
AA3 is absent or is a naturally occurring amino acid in the D or L configuration, which is selected from the group consisting of alanine, phenylalanine, serine, isoleucine, norvaline, S-methylcysteine, methionine, glutamate, histidine, glycine, aspartate, tryptophane, and leucine, and can optinally carry protective groups,
AA4 is absent or is a naturally occurring amino acid in the D or L configuration, which is selected from the group consisting of glycine, leucine, cysteine and norvaline, and can optionally carry protective groups,
AA5 is absent or is a naturally occurring amino acid in the D or L configuration, which is selected from the group consisting of glycine, alanine, valine, leucine, histidine, glutamine, phenylalanine, isoleucine, and methionine,
AA6 is absent or is a naturally occurring amino acid in the D or L configuration, which is selected from the group consisting of glycine, proline, glutamine, methionine, and leucine;
AA7 is absent or is a naturally occurring amino acid in the D or L configuration, which is selected from the group consisting of glycine, leucine, aspartate, histidine, γ-aminobutyric acid and proline;
AA8 is absent or is a naturally occurring amino acid in the D or L configuration, which is selected from the group consisting of glycine, proline and γ-aminobutyric acid ;
and the other radicals CT, Sp1, Sp2 and K are as defined in claim 1.

4. Conjugate according to claim 2 or 3, **characterized in that**
CT is camptothecin or a camptothecin derivative, which can be bonded to the rest of the conjugate via the C20-OH group,
LI is as defined in claim 2 or 3;
Sp is absent, or is a carbonyl or a thiocarbonyl radical,
K is a carbohydrate moeity of the formula (II) 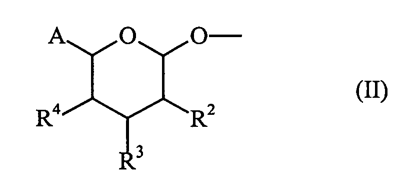 wherein
A is methyl, hydroxymethyl, carboxy and esters und amides derived therefrom, alkoxymethyl, acyloxymethyl oder carboxyalkyloxymethyl and esters und amides derived therefrom, or CH₂-B,
wherein
B is also a carbohydrate radical of the formula (II) which is bonded via its anomeric centre;
R², R³ and R⁴ are identical or different from each other and denote H, hydroxy, alkyloxy, carboxyalkyloxy and esters und amides derived therefrom, hydroxyalkyloxy, aminoalkyloxy, acyloxy, carboxyalkylcarbonyloxy, sulfato, phosphato, halogen, or a modified carbohydrate radical of the formula (II) which is bonded via its anomeric centre, wherein R2 additionally can denote amino or acylamino, and/or wherein two of the radicals R², R³ and R⁴ together can form an epoxy moiety.

5. Conjugate according to Claim 2 or 3, **characterized in that**
Sp2 is arylene which is substituted in ortho, meta or para position with K and Sp1 and can additionally carry 1 to 4 further radicals which are identical or different from each other and are selected from the group consisting of H, methyl, methoxy, hydroxy, carboxy, methyloxycarbonyl, cyano, nitro, halogen, sulfonyl oder sulfonamide,
or is a linear or branched alkylene radical,
and the other radicals CT, LI, Sp1 and K are as defined in claim 2 or 3.

6. Conjugate according to any of the claims 1 to 5, **characterized in that**
CT is camptothecin, which can be linked to the rest of the conjugate via the C20-OH group;
and the other radicals LI, Sp1, Sp2, and K are as defined in claims 1 to 5.

7. Process for the preparation of conjugates according to Claim 1, comprising the reaction of a compound of the formula (III)
K-Sp2-NH₂ (III)
wherein K and Sp2 are as defined in claim 1,
with a carbonic acid derivative such as, for example, phosgene, thiophosgene or a chloroformic acid ester, if appropriate in the presence of a base,
followed by the reaction with a compound of the formula (IV) which has a free primary or secondary amino group
CT-LI (IV)
wherein CT and LI are as defined in claim 1,
and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid.

8. Compound according to any of the claims 1 to 6 for the treatment of diseases.

9. Medicament, comprising at least one of the compounds according to one of Claims 1 to 6.

10. Use of compounds according to one of Claims 1 to 6 for the production of medicaments for the treatment of carcinomatous disorders.
